# EUROPÄISCHE PATENTSCHRIFT

(11) **EP 1 791 950 B1**
(45) Veröffentlichungstag und Bekanntmachung des Hinweises auf die Patenterteilung: **10.01.2018**
(21) Anmeldenummer: 05783928.4
(22) Anmeldetag: 07.09.2005
(51) Int. Cl.: C12N 5/07, C12N 5/0735

(54) **SKALIERBARER PROZESS ZUR KULTIVIERUNG UNDIFFERENZIERTER STAMMZELLEN IN SUSPENSION**
SCALABLE PROCESS FOR CULTIVATING UNDIFFERENTIATED STEM CELLS IN SUSPENSION
PROCESSUS EVOLUTIF POUR CULTIVER DES CELLULES SOUCHES INDIFFERENCIEES EN SUSPENSION

(30) Priorität: 07.09.2004 DE 102004043256
(43) Veröffentlichungstag der Anmeldung: 06.06.2007
(73) Patentinhaber: Rheinische Friedrich-Wilhelms-Universität Bonn, 53113 Bonn (DE)
(72) Erfinder: TERSTEGGE, Stefanie, 53105 Bonn (DE); BRÜSTLE, Oliver, 53105 Bonn (DE)
(74) Vertreter: Grund, Martin
(86) Internationale Anmeldenummer: PCT/EP2005/009611
(87) Internationale Veröffentlichungsnummer: WO 2006/027229

(56) Entgegenhaltungen:
- DANG STEPHEN M ET AL: "Controlled, scalable embryonic stem cell differentiation culture" STEM CELLS (MIAMISBURG), Bd. 22, Nr. 3, 2004, Seiten 275-282, XP002362423 ISSN: 1066-5099
- ANONYMOUS: "cellferm produkte" INTERNET ARTICLE, [Online] XP002362424 Gefunden im Internet: URL:http://www.dasgip.de/de/prozess/kultur /produkte/cellferm.php3>
- GERECHT-NIR SHARON ET AL: "Bioreactor cultivation enhances the efficiency of human embryoid body (hEB) formation and differentiation" BIOTECHNOLOGY AND BIOENGINEERING, Bd. 86, Nr. 5, 5. Juni 2004 (2004-06-05), Seiten 493-502, XP002362425 ISSN: 0006-3592 & US 2003/017589 A1 (MANDALAM RAMKUMAR ET AL) 23. Januar 2003 (2003-01-23)
- NEWMAN K D ET AL: "Poly(d,l lactic-co-glycolic acid) microspheres as biodegradable microcarriers for pluripotent stem cells" BIOMATERIALS, ELSEVIER SCIENCE PUBLISHERS BV., BARKING, GB, Bd. 25, Nr. 26, November 2004 (2004-11), Seiten 5763-5771, XP004508622 ISSN: 0142-9612

## Beschreibung

### Stand der Technik

Die Herstellung von Ersatzgewebe in Zellkultur, das sogenannte Tissue Engineering, die Bereitstellung von Surrogatzellen für zellbasierte Transplantationstherapien und die Identifizierung neuer Wirkstoffe mit Hilfe zellbasierter Hochdurchsatzverfahren sind Paradebeispiele für innovative Anwendungen der Biotechnologie. Hierbei sind Stammzellen eine besonders geeignete Quelle, um solche Biotechnologieanwendungen zu ermöglichen, denn im Gegensatz zu primären Zellen können Stammzellen als potentiell unbegrenzte Quelle für die Gewinnung differenzierter, spezifischer Zelltypen dienen. Eine Kultivierung insbesondere humaner embryonaler Stammzellen unter kontrollierten Bedingungen ist jedoch nach dem Stand der heutigen Technik nicht möglich. Die bislang verwendeten Kulturbedingungen sind durch das Fehlen einer Skalierbarkeit des Prozesses, die Abhängigkeit von einem Ko-Kultur-System und eine mangelnde Kontrolle der Prozessparameter gekennzeichnet. Eine skalierbare Suspensionskultur embryonaler Stammzellen ist bisher nur an differenzierenden Zellen, sogenannten Embryoidkörpern, gezeigt worden. Im Einzelnen wurde eine Suspensionskultur embryonaler Stammzellen in Form von Embryoidkörpern bislang in einem rotating-wall-Bioreaktor (Gerecht Nir et al., Biotechnol Bioeng. 2004, 86(5): 493-502) sowie in gerührten Systemen (Zandstra et al., Tissue Eng. 2003, 9: 767-778; Dang et al., Stem Cells 2004, 22(3):2 75-82; Wartenberg et al. Lab Invest. 1998 Oct; 78(10): 1301-14) etabliert. Dabei ermöglicht der rotating-wall-Bioreaktor bei der Differenzierung der Stammzellen zu gewebeähnlichen Aggregaten eine organotypische Kultur, weist jedoch den Nachteil einer mangelnden Skalierbarkeit zu größeren Volumina auf.

Dang et al. (Stem Cells 2004, 22(3):2 75-82) beschreiben Wachstums- und Vermehrungsexperimente mit eingekapselten Stammzellen aus der Maus. Die Zellen werden in Suspension in Bioreaktoren kultiviert

US2003/017589 beschreibt ein System zur Kultur humaner pluripotenter Stammzellen ohne die Notwendigkeit zur Ko-Kultur.

Allen diesen publizierten Kultivierungsmethoden zu Eigen ist jedoch die Beschränkung des Prozesses auf differenzierende oder differenzierte Stammzellen. Eine Kultur undifferenzierter, pluripotenter Zellen ist unter diesen Kulturbedingungen nicht realisierbar. US 2003/017589 beschreibt ein System zur Kultur humaner pluripotenter Stammzellen ohne die Notwendigkeit zur Ko-Kultur. Die Kultivierung undifferenzierter Stammzellen aus Primaten erwies sich bislang generell als sehr aufwändig und gegenüber äußeren Einflüssen anfällig.

### Zusammenfassung der Erfindung

Diese Erfindung hat die Aufgabe, ein Verfahren zur Kultivierung undifferenzierter Stammzellen bereitzustellen. Eine weitere Aufgabe der Erfindung ist es, einen skalierbaren und kontrollierbaren Prozess zur Kultivierung dieser Zellen zu ermöglichen.

Die Lösung dieser Aufgaben ergibt sich aus den Patentansprüchen. Die Erfindung betrifft ein Verfahren zur Vermehrung von Stammzellen umfassend das Einbringen von Medium, Microcarriern und Stammzellen in ein Gefäß, die Kultivierung von Stammzellen in diesem Gefäß, wobei die Zellen nach Beginn der Kultivierung ihre Stammzelleigenschaften nicht verlieren, und die Entnahme von Zellen aus dem Gefäß, wobei nach Beginn der Kultivierung das Medium abwechselnd für zwischen 10 und 100 Minuten bewegt wird und zwischen 10 und 60 Minuten nicht bewegt wird.

Verschiedene Arten von Gefäßen, wie Spinner-Flaschen, geregelte oder ungeregelte Bioreaktoren, sind für das Verfahren geeignet. Das Medium in diesen Gefäßen kann bewegt werden, wobei das Bewegen des Medium durch einen Rührer erfolgen kann. Als Medium kann definiertes Medium verwendet werden. Das Medium kann auch konditioniert werden, indem es mit anderen, von Stammzellen verschiedenen, Zellen präinkubiert wird. Solche von Stammzellen verschiedene Zellen können murine oder humane Fibroblasten sein. Die Microcarrier, die in dem erfindungsgemäßen Verfahren Verwendung finden, können aus einem einheitlichen Material oder aus einem Trägermaterial und einer Beschichtung bestehen. In dem erfindungsgemäßen Verfahren können je nach Volumen der Kultur Gefäße verschiedener Größe, z.B. Gefäße, die mindestens 10 oder 100 Liter fassen, verwendet werden. Die Entnahme von Microcarriern und Medium aus dem Gefäß kann laufend erfolgen, wobei entnommene Microcarrier und entnommenes Medium durch frische Microcarrier und frisches Medium ersetzt werden. Eine bevorzugte Ausführungsform des erfindungsgemäßen Verfahrens zeichnet sich dadurch aus, dass die eingebrachten Stammzellen ihre Stammzelleigenschaften nach Beginn der Kultivierung nicht verlieren. Eine weitere Ausführungsform ist dadurch gekennzeichnet, dass sich die Anzahl der Zellen gegenüber der Ausgangszellzahl erhöht.

### Definitionen

### Sauerstoffpartialdruck:

Der Sauerstoffpartialdruck im Sinne der Erfindung ist der Anteil des Sauerstoffs an einem Luftgemisch, bei dem eine Kultivierung der Zellen stattfindet. Bei einer Begasung eines Kulturgefäßes unter einem atmosphärischen Sauerstoffpartialdruck von 21% stellt sich bei 37°C in dem wässrigen Medium eine Gelöstsauerstoffkonzentration von 100% ein.

### Stammzellen:

Im Gegensatz zu primären Zellen können Stammzellen als potentiell unbegrenzte Quelle für die Isolierung differenzierter, spezifischer Zelltypen dienen (Poulsom R. et al., J Pathol. 2002 Jul;197(4):441-56; Gepstein L. Circ Res. 2002 Nov 15;91(10):866-76). Unter Stammzellen sind im Kontext dieser Erfindung Zellen gemeint, die sich zum Einen selbst erneuern können, dass heißt unter Aufrechterhaltung ihrer Stammzelleigenschaften vermehrt werden können, und zum Anderen in einem als Differenzierung bezeichneten Vorgang einen oder mehrere spezialisierte Zelltypen, wie beispielsweise Herzmuskelzellen, Endothelzellen, Knorpelzellen, Knochenzellen, Fettzellen, neuronale Zellen, Leberzellen oder Insulin bildende Zellen bilden können (Wobus AM. Mol Aspects Med. 2001 Jun; 22(3): 149-64).

Beispiele für Stammzellen sind Stammzellen aus Nabelschnurblut (Sanchez-Ramos JR., J Neurosci Res. 2002 Sep 15;69(6):880-93.), neuronale Stammzellen (Hitoshi S. et al., Genes Dev. 2002 Apr 1;16(7):846-58. Okano H. J Neurosci Res. 2002 Sep 15;69(6):698-707.), mesenchymale Stammzellen aus dem Knochenmark oder dem peripheren Blut (Jiang Y. et al., Exp Hematol. 2002 Aug;30(8):896-904; Schwartz et al., J Clin Invest. 2002 May;109(10):1291-302) sowie Stammzellen aus der Haut (Toma et al., Nat Cell Biol. 2001 Sep;3(9): 778-84), dem Pankreas (Means AL., Pancreatology. 2001;1(6): 587-96), der Leber (Suzuki A., et al., Cell Transplant. 2001;10(4-5):393-6), dem Darm (Brittan M., J. Pathol. 2002 Jul;197(4):492-509) oder aus Fettgewebe (Cannon B. et al., Methods Mol Biol. 2001;155:213-24), um nur einige zu nennen. Weitere wichtige Stammzellen sind die embryonalen Keimzellen (Schamblott MI., et al., Proc Natl Acad Sci U S A. 2001 Jan 2;98(1): 113-8), die embryonalen Karzinomzellen (Friedrich, T.D. et al., Differentiation. 1983; 24, 60-64; Andrews, P.W., Biochim. Biophys. Acta. 1988; 948, 17-36) sowie die sogenannten embryonalen Stammzellen (ES), die aus der inneren Zellmasse einer Blastozyste isoliert werden können. Embryonale Stammzellen wurden bereits aus einer Vielzahl von Spezies etabliert, unter anderem aus der Maus (Evans MJ, Kaufman MH., Nature. 1981 Jul 9; 292(5819):154-6) und dem Menschen (hier nur als Referenzdokument zitiert: Thomson JA, et al., Science. 1998 Nov 6;282(5391): 1145-7). ES sind pluripotente Stammzellen, das heißt sie können in eine Vielzahl spezifischer Zelltypen differenzieren (Wobus AM., Mol Aspects Med. 2001 Jun;22(3): 149-64; Amit M, Itskovitz-Eldor J., J Anat. 2002 Mar; 200(Pt 3):225-32). Sowohl für humane als auch für murine ESC konnte gezeigt werden, dass sie unter anderem in Herzmuskelzellen (Klug et al. J. Clin. Invest. 1996 Jul.; 98 (1):216-24; Mummary C. et al., J Anat. 2002 Mar;200(Pt 3):233-42.; Xu C. et al., Circ Res. 2002 Sep 20;91(6):501-8), Insulin produzierende Zellen (Assady et al., Diabetes. 2001 Aug;50(8):1691-7; Soria B. Differentiation. 2001 Oct;68(4-5):205-19.; Lumelsky M. et al,. Science. 2001 May 18;292(5520):1389-94), neurale Vorläufer und neuronale Zellen (Schuldiner M. et al., Brain Res. 2001 Sep 21;913(2):201-5); Brüstle, O. et al, Science. 1999 Jul 30;285(5428):754-6); Okabe S, et al, Mech Dev. 1996 Sep;59(1):89-102; Zhang SC et al, Nat Biotechnol. 2001 Dec;19(12):1129-33), Endothelzellen (Levenberg S. Proc Natl Acad Sci USA. 2002 Apr 2;99(7):4391-6) und blutbildende Zellen (Kaufmann DS., Proc Natl Acad Sci USA. 2001 Sep 11;98(19):10716-21) differenzieren können, um nur einige zu nennen. Bei Stammzelllinien handelt es sich um ES-Zellen, die in Zellkultur propagiert wurden.

### Somatische Zellen:

Mit "somatischen Zellen" sind solche Zellen gemeint, die in einen oder mehrere gewebespezifische Zelltypen ausreifen können oder bereits ausgereift sind. Somatische Zellen können die folgenden Gewebe bilden: Knochen, Zahngewebe, Knorpel, Sehne, Knochenmarksstroma, Nervengewebe, Haut, Pankreas, Leber, Fettgewebe, Muskel und andere.

### Feederzellen:

Feederzellen sind Zellen eines Typs, die für die Kultivierung eines zweiten Zelltyps erforderlich sind. Im Zusammenhang mit der Kultivierung von ES-Zellen haben Feederzellen die Funktion, das Überleben, die Proliferation und die Pluripotenzerhaltung der ES-Zellen zu sichern.

Dies kann durch direkte Kokultivierung der Zellen des ersten und zweiten Typs ermöglicht werden oder durch Kultivierung der Zellen des ersten Typs in einem Medium, das dann den Zellen des zweiten Typs zur Verfügung gestellt wird, nachdem die Zellen des ersten Typs für eine bestimmte Zeit in dem Medium kultiviert worden waren. Vor der Übertragung des Mediums von den Zellen des ersten Typs auf die Zellen des zweiten Typs werden möglichst alle Zellen des ersten Typs aus dem Medium entfernt. Die Feederzellen können vor Beginn der Kultivierung bestrahlt oder mit Mitomycin C behandelt werden, um ein weiteres Wachstum dieser Zellen zu vermeiden. In einer bevorzugten Ausführungsform werden die Feederzellen mit Gammastrahlen bestrahlt.

### Gefäße:

Für das erfindungsgemäße Verfahren sind als Gefäße Bioreaktoren, Spinner und andere zur Kultivierung von Zellen geeignete Gefäße geeignet. Unter einem Bioreaktor ist in dieser Erfindung ein Behältnis gemeint, das zur Kultivierung eukaryontischer Zellen, vorzugsweise tierischer Zellen, besonders bevorzugt zur Kultivierung von Säugerzellen geeignet ist.

Unter einem geregelten Bioreaktor sind Bioreaktor-Systeme gemeint, bei denen mindestens der Sauerstoffpartialdruck im Kulturmedium mit geeigneten Geräten gemessen und geregelt werden wird. Geeignete Geräte zur Messung des Sauerstoffpartialdrucks sind beispielsweise Sauerstoffelektroden. Der Sauerstoffpartialdruck kann beispielsweise über die Menge und die Zusammensetzung des verwendeten Gasgemischs (z.B. Luft oder ein Gemisch aus Luft und / oder Sauerstoff und / oder Stickstoff und / oder Kohlendioxyd) geregelt werden. Geeignete Geräte zur Messung und Regelung des Sauerstoffpartialdrucks sind unter Bailey, JE. (Bailey, JE., Biochemical Engineering Fundamentals, second edition, MCGraw-Hill, Inc. ISBN 0-07-003212-2 Higher Education, (1986)) oder Jackson AT. (Jackson AT., Verfahrenstechnik in der Biotechnologie, Springer, ISBN 3540561900 (1993)) beschrieben. Das Kultivierungsvolumen eines geregelten Bioreaktors beträgt zwischen 20 ml und 500 ml. Geeignete Gefäße sind demnach auch Spinner. Spinner sind geregelte oder ungeregelte Bioreaktoren, die gerührt werden können, und bei denen unterschiedliche Rührer, wie Glasball-Rührer, Paddelrührer und andere geeignete Rührer verwendet wird können. Das Kultivierungsvolumen eines Spinners liegt zwischen 20 ml und 500 ml. Rollerflaschen sind runde Zellkulturflaschen aus Plastik oder Glas mit einer Kulturfläche von zwischen 400 und 2000 cm². Die Zellen werden auf der gesamten inneren Oberfläche dieser Flaschen kultiviert, die Benetzung mit Medium wird dabei durch "Rollern", das heißt Drehen der Flaschen um die eigene Achse, gewährleistet.

### Microcarrier:

Erfindungsgemäße Microcarrier (oder Carrier) sind Trägermaterialien, vorzugsweise in Kugelform, für die Kultivierung adhärent wachsender Zellen, insbesondere tierischer Zellen in Suspension. Microcarrier sind auch im IUPAC Compendium of Chemical Terminology (2nd Edition 1992, Vol. 64, S. 160) definiert. Erfindungsgemäße Microcarrier sind insbesondere Trägermaterialien, vorzugsweise in Kugelform, für die Kultivierung von Stammzellen in Suspension. Microcarrier bestehen vorzugsweise aus einem Material ausgewählt aus der Gruppe bestehend aus Plastik, Glas, Keramik, Silikon, Gelatine, Dextran, Cellulose und anderen. Zudem können Microcarrier auf unterschiedliche Weise vorbehandelt (z.B. Plasmabehandlung von Plastikoberflächen, die zur Hydrophilierung der Oberfläche führt) oder beschichtet sein (z.B. mit Gelatine, Fibronectin, Laminin, Polyornithin, Matrigel oder mit Bindemotiven wie der RGD-Bindedomäne von Fibronectin und anderen). Kommerziell erhältliche Microcarrier sind Cytodex 1, Cytodex 3, Cytopore (Amersham Biosciences), Cultispher G, Cultispher S (Perbio), Pronectin, FACT (Sigma), Biosilon, Microhex 2D (Nunc) und ImmobaSil (Dunn), und andere geeignete Microcarrier.

### Medien:

Medien sind Flüssigkeiten, die Bestandteile enthalten, die die Kultivierung von Zellen ermöglichen und/oder die Proliferation fördern. Ein Medium kann jegliche der im Folgenden genannten Bestandteile in geeigneter Menge und Konzentration enthalten: Isotonische Salzlösungen, Puffer (z.B. Bicarbonat/CO₂), Aminosäuren, eine oder mehrere Kohlenstoffquellen (z.B. Glucose), Antibiotika, Serum oder Stoffe, die das Serum ersetzen, Wachstumsfaktoren, Hormone (z.B. Insulin), Vitamine, Spurenelemente, Albumin, Transferrin, Lipide und Phenolrot als pH-Indikator. In bevorzugten Ausführungsformen wird kommerziell erhältliches Medium wie Dulbecco's modified Eagle's medium (DMEM) verwendet. In bevorzugten Ausführungsformen werden DMEM oder andere Medien verwendet, die für die Kultivierung von Stammzellen besonders geeignet sind. Dazu zählen Knockout-DMEM und andere Medien. Knockout DMEM ist ein Medium optimiert für die Kultivierung von undifferenzierten Stammzellen (beschrieben in Goldsborough, M., et al. (1998), Focus, 20, 8).

### Konditioniertes Medium:

Konditioniertes Medium für die Kultivierung von ES-Zellen kann aus Knockout-DMEM mit 20% Serum Replacement, 1% nicht-essentiellen Aminosäuren, 0,5% L-Glutamin und 0,1mM β-Mercaptoethanol bestehen, das für 24 Stunden von einem dazu geeigneten Zelltyp konditioniert wurde. Vor dem Gebrauch wird das Medium filtriert und mit 4 ng/ml bFGF versetzt. Zur Konditionierung geeignet sind etwa primäre Fibroblasten der Maus, menschliche Fibroblasten und Fibroblastenzelllinien. Diese Zellen können sich im Wachstum befinden oder durch Gamma-Bestrahlung oder eine andere geeignete Behandlung mitotisch inaktiviert sein. Zwischen 100000 und 200000, bevorzugt 140000 Zellen pro ml werden für die Konditionierung eingesetzt. Die Konditionierung kann in jedem Zellkulturgefäß erfolgen, auch in einem Carrier-basierten Bioreaktorprozess.

Das für die Konditionierung eingesetzte Medium kann auch Serum-haltig sein, ebenso ist es denkbar, ein definiertes Medium wie Ex-Vivo (Cambrex) einzusetzen.

### Stammzellmedium:

Das Stammzellmedium kann konditioniertes Medium bestehend aus Knockout-DMEM mit 20% Serum Replacement (Goldsborough, M., et al. (1998), Focus, 20, 8), 1% nicht-essentiellen Aminosäuren, 0,5% L-Glutamin und 0,1mM β-Mercaptoethanol sein, das für 24 Stunden von einem dazu geeigneten Zelltyp konditioniert wurde. Vor dem Gebrauch wird das Medium filtriert und mit 4 ng/ml fibroblast growth factor (bFGF) versetzt.

In einer weiteren Ausführungsform wird ein ansonsten identisches Medium, das statt 20% Serum Replacement 20% Fetal Calf Serum (FCS, Fötales Kälberserum) enthält, eingesetzt.

Möglich ist auch der Einsatz von Knockout-DMEM mit 15% Serum Replacement, 1% nicht-essentiellen Aminosäuren, 2mM L-Glutamin, 0,1mM β-Mercaptoethanol, 4ng/ml basic fibroblast growth factor (bFGF), 0,12ng/ml tumor growth factor β1 (TGFβ1) und 1000U/ml leukemia inhibitory factor protein (LIF) oder von ExVivo (Cambrex) mit 40 ng/ml bFGF und 15 ng/ml flt3-Ligand (FMS-like tyrosine kinase 3-ligand).

### Nachweis von Pluripotenzmarkern:

Menschliche undifferenzierte embryonale Stammzellen exprimieren SSEA-3, SSEA-4, Tra-1-60, Tra-1-81, OCT-4, hTERT, Nanog, Cripto und andere sogenannte Stammzellmarker (diskutiert in: Brivanlou AH et al., Science. 2003 May 9; 300(5621): 913-6; Henderson JK et al., Stem Cells. 2002; 20(4):329-37 und Ben-Shushan, E. et al. 1998; Mol. Cell Biol. 18, 1866-1878). All diese Marker sind geeignet zum Nachweis des Stammzellcharakters der Zellen. Die Expression dieser Marker kann mittels Immunfluoreszenz, FACS-Analyse, *In Situ* Hybridisierung, Reverse Transkriptase Echtzeit PCR, Northern Blot oder Western Blot erfolgen.

Beispielsweise können zum Nachweis der Oct4-Expression die Zellen mit PBS gewaschen, für 10 Minuten mit 4% Paraformaldehyd (PFA) fixiert, mit PBS gewaschen und für 20 Minuten mit 0,5% Triton permeabilisiert werden. Durch Blocken mit 5% Normal Goat Serum (NGS) und 0.1% Triton für 60 Minuten wird eine unspezifische Bindung der Antikörper verhindert. Die Inkubation mit dem ersten Antikörper (anti-Oct4-rabbit-IgG, Santa Cruz Biotechnology Inc., 1:400 in 5% NGS + 0,1% Triton verdünnt) erfolgt über Nacht bei Raumtemperatur. Anschließend wird mit PBS gewaschen und für 60 Minuten bei Raumtemperatur mit dem sekundären Antikörper (anti-rabbit-IgG-Cy3, Jackson Immuno Research, 1:200 in 5% NGS + 0,1% Triton verdünnt) inkubiert. Nach erneutem Waschen in PBS erfolgt eine 2minütige Inkubation mit DAPI (Sigma) in einer 1:10000 Verdünnung und ein weiterer Waschschritt in PBS.

### Antikörper:

Der Ausdruck "Antikörper" bezieht sich auf polyklonale und monoklonale Antikörper. Auch Fragmente und Derivate dieser Antikörper können in der Erfindung Verwendung finden.

### Generelle Techniken und Chemikalien:

Generelle Methoden der Molekularbiologie, der Zellbiologie, der Proteinchemie und zum Thema Antikörpertechniken sind beschrieben in den jeweils aktuellen Ausgaben von "Molecular Cloning: A Laboratory Manual, (Sambrook et al., Cold Spring Harbor)"; Current Protocols in Molecular Biology (F.M. Ausubel et al. Eds., Wiley & Sons); Current Protocols in Protein Science (J.E. Colligan et al. eds., Wiley & Sons); Current Protocols in Cell Biology (J.S. Bonifacino et al., Wiley & Sons) und Current Protocols in Immunology (J.E. Colligan et al., eds Wiley & Sons).

Generelle Methoden zum Thema Zellkultur und Medien sind beschrieben in "Large Scale Mammalian Cell Culture (Hu et al., Curr. Opin., Biotechnol. 8: 148, 1997); "Serum free Media" (K. Kitano, Biotechnol. 17: 73, 1991) und "Suspension Culture of Mammalian Cells" (Birch et al. Bioprocess Technol. 19: 251, 1990),

Methoden zum Thema Stammzellen sind beschrieben in "Teratocarcinoma and embryonic stem cells: A practical approach" (E. J. Robertson, ed., Press Ltd, 1987); "Guide to Techniques in Mouse Development" (P. M. Wassermann et al. eds., Academic Press, 1993); "Embryonic Stem Cell Differentiation in Vitro" (M.V. Wiles, Meth. Enzymol. 225: 900, 1993); "Properties and uses of Embryonic Stem Cells: Prospects for Application to Human Biology and Gene Therapy" (P.D. Rathjen et al., 1993); "Embryonic Stem Cells, Methods and Protocols" (K. Turksen ed., Humana Press, 2002) und "Human Embryonic Stem Cells" (A. Chiu und M.S. Rao, Humana Press, 2003). Ein Überblick über die Diffenzierung von Stammzellen wird gegeben in Robertson, Meth. Cell Biol. 75: 173, 1997 und Pedersen, Reprod. Fertil. Dev. 10: 31, 1998.

Methoden der Bioprozesstechnik sind beschrieben in "Bioprozesstechnik" (H. Chmiel Hrsg., Gustav Fischer Verlag 1991); "Bioreaktoren und periphere Einrichtungen. Ein Leitfaden für die Hochschulausbildung, für Hersteller und Anwender" (Winfried Storhas, Springer Verlag 1994); "Bioprocess Engineering Principles" (Pauline M. Doran, Academic Press 1997) und "Bioprocess Engineering: Basic Concepts" (Michael L-Shuler, Prentice Hall 2000).

Reagenzien, Medien und Kits können von jedem kommerziellen Anbieter, der diese zur Verfügung stellt, bezogen werden, wie Sigma, BioRad, Stragene, Roche und weitere.

### Die mittlere spezifische Wachstumsrate:

Die mittlere spezifische Wachstumsrate berechnet sich nach folgender Differentialgleichung, wobei µ die spezifische Wachstumsrate, x die Zellzahl und t die Zeit sind: dx/dt = µx.

### Beschreibung der Erfindung

Die Erfindung offenbart einen technischen Prozess, der es ermöglicht, Stammzellen auf Microcarriern in Bioreaktoren zu kultivieren und zu vermehren. Damit wird erstmals ein Verfahren vorgestellt, das skalierbar ist, also eine nahezu beliebige Vergrößerung des Kultivierungsansatzes ermöglicht. Nach der Kultivierung können die Zellen durch geeignete Bedingungen zur Differenzierung in alle somatischen Zelltypen stimuliert werden. Durch das erfindungsgemäße Verfahren ist es möglich, große Mengen an Stammzellen zu kultivieren, wie sie für die Erstellung von Ersatzgewebe benötigt werden. Dieses Verfahren findet vorzugsweise in Bioreaktoren statt, in denen es durch Regelung möglich ist, die gewünschten Kulturbedingungen in Hinsicht auf Temperatur, pH-Wert, Sauerstoffgehalt und andere Parameter genau einzustellen. Diese exakte Regelung ist am besten in einem Bioreaktor möglich und erlaubt die Kultivierung der Stammzellen unter genau definierten Bedingungen. Genau definierte Bedingungen sind notwendig, um den Stammzellcharakter der Zellen so weit wie möglich erhalten zu können und die Differenzierung zu somatischen Zelltypen zu vermeiden.

### Einbringen der Zellen

In einer Ausführungsform der Erfindung können die Stammzellen in das Gefäß eingebracht werden, nachdem das Gefäß mit Medium und Microcarriern befüllt wurde. In einer weiteren Ausführungsform werden die Stammzellen zunächst mit den Microcarriern in Medium vorinkubiert. Es findet demnach eine Vorkultivierung statt, die den Zweck hat, eine Anheftung der Zellen an die Microcarrier zu ermöglichen. Diese Vorkultivierung kann im eigentlichen Kultivierungsgefäß erfolgen oder in einem Gefäß, das verschieden ist von dem Gefäß zur eigentlichen Kultivierung. Dies kann in jedem für die Vorkultivierung geeigneten Gefäß erfolgen. Während der Vorkultivierung wird das Medium nicht bewegt. Insbesondere Zellkulturflaschen sind dazu geeignet, wie sie für die Kultivierung von Zellen in Suspension verwendet werden. Nach Anheftung der Stammzellen an die Microcarrier werden die mit Stammzellen beladenen Microcarrier in das Gefäß für die eigentliche Kultivierung, die Hauptkultivierung, eingebracht oder, wenn die Vorkultivierung bereits im gleichen Gefäß erfolgte wie die Hauptkultivierung, damit begonnen, das Medium im Gefäß zu bewegen. Eventuell kann nun noch zusätzliches Medium hinzugefügt werden. Auch ist es möglich, zusätzlich noch nicht beladene Microcarrier in das Gefäß einzubringen. Nach Einfüllen der beladenen oder unbeladenen Microcarrier in das Gefäß beginnt die Kultivierung.

In einer besonders bevorzugten Ausführungsform sind die Stammzellen embryonale Stammzellen. Besonders bevorzugt ist die Verwendung von Stammzellen, die embryonale Keimzellen sind, die embryonalen Karzinomzellen sind, sowie die Verwendung der sogenannten embryonalen Stammzellen (ES). In einer weiteren Ausführungsform werden Stammzellen verwendet, die aus Nabelschnurblut gewonnen wurden, oder es werden verwendet neuronale Stammzellen, mesenchymale Stammzellen aus dem Knochenmark oder Stammzellen, die aus dem peripheren Blut gewonnen wurden, sowie Stammzellen aus der Haut, oder Stammzellen, die aus dem Pankreas, der Leber, dem Darm oder aus Fettgewebe gewonnen wurden. In einer bevorzugten Ausführungsform sind die Stammzellen adulte Stammzellen.

### Entnahme von Zellen

Ein Vorteil des erfindungsgemäßen Verfahrens liegt in der Möglichkeit, jederzeit Microcarrier mit Zellen aus dem Gefäß zu entnehmen. Dies geschieht durch steriles Absaugen oder Abnehmen von Medium mit den darin enthaltenen Carriern. Dies kann zu Kontrollzwecken erfolgen, um die Stammzelleigenschaften der Zellen zu kontrollieren, oder um im Rahmen einer Dauerkultur eine kontinuierliche Produktion von Stammzellen mit gleich hoher Qualität und Quantität für die spätere Differenzierung zu somatischen Zelltypen zu gewährleisten. Die laufende Kontrolle sichert, dass die Charaktereigenschaften der Zellen erhalten bleiben. In zweidimensionalen Systemen ist dies nicht möglich, da die Stammzellen von Kulturschale zu Kulturschale unterschiedliche Eigenschaften aufweisen können. Weiter ist in zweidimensionalen Systemen eine Entnahme eines Teils der Zellen gar nicht oder nur unter erheblicher Störung der verbliebenen Zellen möglich. Mit Hilfe einer Suspensionskultur, wie sie durch das erfindungsgemäße Verfahren ermöglicht wird, können Zellen einer bereits gut laufenden Kultur entnommen werden, ohne die Kultur als Ganzes entnehmen zu müssen. Es ist hingegen technisch nicht vorteilhaft, mit einer Vielzahl verschiedener Kulturschalen, wie in der zweidimensionalen Zellkultur üblich, operieren zu müssen, die jede für sich der Pflege und Kontrolle bedürfen.

Nach einer Entnahme von Microcarriern mit Zellen können neue Microcarrier eingebracht werden, um eine weitere Expansion der Zellen zu ermöglichen. Somit ist die Kontinuität der Kultur gesichert, und ein Kultursatz kann beispielsweise für die Differenzierung zu einer großen Menge verschiedener Zelltypen eingesetzt werden.

Nach der Entnahme von mit Zellen bewachsenen Microcarriern können die Zellen durch Inkubation mit Trypsin (5 Minuten) oder mit Collagenase (2 h) von den Carriern abgelöst werden. Diese enzymatische Behandlung kann auch unter Rühren im Kulturgefäß erfolgen. Die anschließende Trennung der Zellen von den Carriern erfolgt durch sieben (bei Trypsin-Behandlung) oder durch prä-Plattieren der Zellen und anschließendes Abspülen der Carrier (bei Collagenase-Behandlung). Es ist auch möglich, die bewachsenen Carrier auf Zellkulturschalen zu plattieren und die verbleibenden Carrier nach dem Auswachsen der Zellen auf die Zellkulturschale abzuspülen.

Offengelegt wird, dass die Microcarrier mit den Zellen nicht aus dem Kulturgefäß entnommen werden müssen, sondern die Differenzierung der Zellen in dem Gefäß eingeleitet werden kann.

### Phase des Bewegens und Nicht-Bewegens des Mediums und der Zellen

In einer Ausführungsform der Erfindung findet nach erstmaligem Einbringen der Zellen in das Medium des Gefäßes eine Ruhephase statt, die ein Anheften der Stammzellen an die Microcarrier erlaubt. In einer bevorzugten Ausführungsform der Erfindung beträgt die Dauer der Ruhephase maximal einen Tag. In einer besonders bevorzugten Ausführungsform beträgt die Dauer der Ruhephase maximal zwölf Stunden. Nach Anheftung der Zellen wird das Medium abwechselnd bewegt und nicht bewegt. Die Phase der Bewegung ermöglicht den Zellen einen kontinuierlich gleichmäßigen Austausch mit dem sie umgebenden Medium und ermöglicht eine maximale Ausnutzung des im Gefäß vorhandenen Raumes. Außerdem wird durch das Bewegen sicher gestellt, dass überall im Gefäß die gleichen Kulturbedingungen herrschen. Dies ist sehr wichtig für die gleichmäßige Einstellung aller für die Kultivierung relevanten Parameter und stellt einen weiteren Vorteil gegenüber einer zweidimensionalen Kultur dar, die mit einer Vielzahl kleinerer, unverbundener Kulturgefäße durchgeführt werden muss, und somit keine einheitlichen Kulturbedingungen gewährleisten kann.

Jedoch besitzt das Bewegen des Mediums auch den Nachteil, dass Scherkräfte auf die anheftenden Zellen wirken, die infolgedessen von den Microcarriern abfallen können. Diese Zellen sterben in der Folge ab und mindern somit die Effizienz des Verfahrens in Hinblick auf Zellzahl als auch Undifferenziertheitsstatus der Zellen. Erfindungsgemäß wird diesem Problem abgeholfen, in dem das Medium in einem Maße bewegt wird, dass diese Scherkräfte optimiert werden und vor allem dadurch, dass sich Phasen des Bewegens des Mediums mit Phasen des Nichtbewegens abwechseln. Letzteres wird in einer speziellen Ausführungsform als Intervallrühren bezeichnet. In diesen Ruhephasen, d.h. den Phasen des Nichtbewegens, können von Microcarriern abgefallene Zellen wieder an Microcarrier anheften. Es konnte in dieser Erfindung gezeigt werden, dass das Intervallrühren einen sehr positiven Einfluss auf die Überlebensrate der Stammzellen, ihre Proliferation und den Erhalt der Stammzelleigenschaften besitzt.

Das Medium wird abwechselnd für zwischen 10 und 100 Minuten bewegt und zwischen 10 und 60 Minuten nicht bewegt. In einer bevorzugten Ausführungsform wird das Medium abwechselnd für zwischen 15 und 45 Minuten bewegt und zwischen 15 und 45 Minuten nicht bewegt. In einer besonders bevorzugten Ausführungsform wird das Medium abwechselnd für zwischen 20 und 35 Minuten bewegt und zwischen 20 und 35 Minuten nicht bewegt. In der bevorzugtesten Ausführungsform wird das Medium abwechselnd für 30 Minuten bewegt und für 30 Minuten nicht bewegt. Dieses Bewegen kann in Spinnerflaschen oder geregelten Bioreaktoren mit einem Rührer bewirkt werden. Dies kann ein Glasball-, Paddelrührer oder anderer geeigneter Rührer sein. Das Rühren kann in einer Ausführungsform bei einer Geschwindigkeit zwischen 20 und 200 Umdrehungen pro Minute (upm) liegen. In einer bevorzugten Ausführungsform erfolgt das Rühren bei einer Geschwindigkeit zwischen 20 und 80 upm. In der bevorzugtesten Ausführungsform erfolgt das Rühren bei 30 upm.

### Kulturmedium

In einer Ausführungsform zeichnet sich das Verfahren dadurch aus, dass das Medium, bevor die Stammzellen eingebracht werden, konditioniert wird. Konditioniert heißt hierbei, dass in dem Medium von den Stammzellen verschiedene Zellen kultiviert werden, die das sie umgebende Medium derart verändern, dass es möglich ist, Stammzellen darin zu kultivieren. Zellen, die Medium konditionieren, werden auch Feederzellen genannt. Diese von den Stammzellen verschiedenen Zellen werden dabei bevorzugt 1 bis 3 Tage, in einer noch bevorzugteren Ausführungsform 1 Tag darin kultiviert. Nach dieser Konditionierung des Mediums durch Präkultivierung mit von den Stammzellen verschiedenen Zellen wird das Medium entnommen, eventuell noch darin enthaltene Zellen von diesem abgetrennt und in das Gefäß zur Kultivierung der Stammzellen eingebracht. Die Abtrennung der Feederzellen findet bevorzugt über Filter statt. Bevorzugt werden Filter eingesetzt, deren Porengröße kleiner ist die Größe der Feederzellen. Vorzugsweise werden die Feederzellen vor Beginn der Konditionierung des Medium mitotisch inaktiviert, vorzugsweise mit Gamma-Strahlen. Somit wird eine Vermehrung der Feederzellen im Medium verhindert. Geeignete Zellen für die Konditionierung von Medium sind Fibroblasten und andere Zellen, die Medium derart konditionieren, dass Stammzellen darin kultiviert werden können. Die Fibroblasten können murinen oder humanen Ursprungs sein. Die Fibroblasten können genetisch verändert sein, zum Beispiel, um rekombinante Proteine zu exprimieren und in das Kulturmedium abzugeben. Diese rekombinanten Proteine können dazu geeignet sein, die Proliferation der Stammzellen anzuregen und/oder zu verhindern, dass diese differenzieren.

Offengelegt wird, dass die ES-Zellen nach Abschluss der Proliferationsphase, in der die Stammzellen ohne Einbuße ihrer Stammzelleigenschaften vermehrt wurden, nicht aus dem Kultivierungsgefäß entnommen werden müssen, sondern dass bei diesen Zellen eine Differenzierung zu somatischen Zellen eingeleitet werden kann. In einer besonders bevorzugten Ausführungsform werden dazu Proteine eingesetzt, welche die Differenzierung der Zellen auslösen können. Dabei kann es sich um EGF (epidermal growth factor), PDGF (platelet-derived growth factor), Retinsäure, HGF (hepatocyte growth factor), BMPs (bone morphogenetic proteins), und andere handeln. Diese Proteine können durch genetisch veränderte Fibroblasten in das Kulturmedium abgegeben werden.

In einer bevorzugten Ausführungsform der Erfindung enthält das Medium kein fötales Kälberserum. Statt des Kälberserums kann eine Kultivierung mit einem Serumersatz erfolgen. Das Medium kann darüber hinaus oder anstelle der Konditionierung Wachstumsfaktoren, wie bFGF, TGFβ1, LIF, flt3-Ligand, und andere Faktoren enthalten, die die Proliferation der Stammzellen anregen und/oder verhindern, dass diese differenzieren.

Beispiele für verwendbare Medienzusammensetzungen sind etwa: Knockout-DMEM mit 20% Serum Replacement, 1% nicht-essentiellen Aminosäuren, 1mM L-Glutamin, 0,1mM 2-Mercaptoethanol und 4 ng/ml bFGF, das für 24 Stunden von murinen Fibroblasten konditioniert wurde, ExVivo (Cambrex) mit 40 ng/ml bFGF und 15 ng/ml flt3-Ligand oder Knockout-DMEM mit 15% Serum Replacement, 1% nicht essentielle Aminosäuren, 2mM L-Glutamin, 0,1mM 2-Mercaptoethanol, 4ng/ml bFGF, 1000U/ml LIF und 0,12ng/ml TGFβ1.

### Microcarrier

Erfindungsgemäße Microcarrier sind Trägermaterialien, vorzugsweise in Kugelform, für die Kultivierung adhärent wachsender Zellen, insbesondere Tierzellen. Die Microcarrier können aus den verschiedensten Materialien bestehen, wie Plastik, Glas, Keramik, Silikon, Gelatine, Dextran, Cellulose und andere. In einer besonders bevorzugten Ausführungsform der Erfindung sind die Microcarrier aus Gelatine, Dextran oder Cellulose. Diese Materialien können entweder biologisch abbaubar sein oder nicht. Die Microcarrier können aus einem einheitlichen Material oder aus einem Trägermaterial und einer Beschichtung bestehen. Das Trägermaterial kann Gelatine, Dextran, Cellulose oder ein anderes geeignetes Material sein. Die Beschichtung kann aus Proteinen oder aus Proteoglykanen der extrazellulären Matrix von Säugetierzellen bestehen. Zur Beschichtung sind alle Proteine, modifizierten Derivate von Proteinen oder Kombinationen davon geeignet, die eine Anheftung der Zellen ermöglichen, insbesondere Proteine, die eine biologische Rolle im Zell-Zellkontakt besitzen, wie Laminin, Fibronectin, Heparin-Sulfat, Kollagen, Matrigel und andere geeignete Proteine. Die Derivate können durch begrenzte Proteolyse, Phosphorylierung, Glykosilierung, genetische Modifikation, Alkylisierung, und andere geeignete Methoden erstellt werden. Auch Gelatine kann zur Beschichtung eingesetzt werden. Die Microcarrier können insbesondere aus kommerziell erhältlichen Materialien wie Cytodex 3, Cultispher G und anderen Materialien bestehen.

Die Beschichtung der Carrier kann durch eine Inkubation des Trägermaterials unter Bewegung in einer Lösung des entsprechenden Proteins erfolgen. Beispielsweise kann das Trägermaterial für 15 bis 60 Minuten in einer 0,1%igen Gelatine-Lösung oder für 2 Stunden in 1:30 in Knockout-DMEM verdünntem Matrigel (eine solubilisierte Basalmembran-Präparation, die aus EHS-Sarkomen der Maus gewonnen wird. Die Haupt-Komponenten sind Laminin, Kollagen IV, Heparin-Sulfat und Entaktin. Hersteller: Becton Dickinson) gerührt werden. Anschließend wird die Lösung entfernt und die Zellen und das Medium werden zugegeben.

Die Dichte der Carrier kann zwischen 0,9 und 1,1 g/ml liegen. Der mittlere Durchmesser d₅₀ der Carrier beträgt zwischen 125 und 300 µm. Die Carrier können solide oder porös sein, dabei kann die Porengröße zwischen 10 und 30 µm liegen.

Je nach Carriertyp werden zwischen 1 g pro Liter Kulturvolumen und 40 g pro Liter Kulturvolumen Carrier für die Kultivierung eingesetzt. Liegt ein nicht geregelter Prozess vor, so sollten 10 g pro Liter Kulturvolumen Carrier nicht überschritten werden.

### Skalierbarkeit des Verfahrens

Ein wichtiger Aspekt dieser Erfindung ist die Möglichkeit, durch das dargestellte Verfahren den Prozess der Kultivierung von Stammzellen skalierbar zu gestalten. Daher ist eine bevorzugte Ausführungsform ein Verfahren, bei dem das Gefäß mindestens 5 Liter umfasst. Bei der bevorzugtesten Ausführungsform umfasst das Gefäß 100 Liter. Das erfindungsgemäße Verfahren erlaubt weiterhin, die Zellen in einem kleineren Start-Kulturvolumen von unter 1 Liter anzuziehen. Kurz bevor die Zellen die Microcarrier vollständig besiedeln, wird das Kulturvolumen vergrößert, indem neues zusätzliches Medium und/oder Microcarrier dem Kulturvolumen hinzugefügt werden. Dabei kann ein Anziehen der Kultur in einem Gefäß erfolgen, das die Größe des Kulturvolumens übersteigt, so dass nur neues Medium und/oder neue Microcarrier hinzugefügt werden können. In einer anderen Ausführungsform entspricht das Volumen des Gefäßes dem Kulturvolumen, und eine Aufstockung des Ansatzes geschieht durch Überführen des Kulturvolumen in ein neues größeres Gefäß, in das neues Medium und/oder neue Microcarrier hinzugefügt oder vorgelegt werden. Bevorzugte Ausführungsformen der Erfindung sind insbesondere Vergrößerungen des Kulturvolumens um das zweifache. Besonders bevorzugt ist eine Ausführungsform der Erfindung, bei der das Kulturvolumen um das zehnfache erhöht wird.

### Zeiträume der Kultivierung

Das erfindungsgemäße Verfahren ermöglicht insbesondere, die Kultivierung über sehr lange Zeiträume durchzuführen, ohne dass die Zellen ihre Stammzelleigenschaften verlieren. In einer Ausführungsform beträgt dieser Zeitraum mindestens zwei Wochen. In einer stärker bevorzugten Ausführungsform beträgt dieser Zeitraum mindestens vier Wochen. In einer besonders bevorzugten Ausführungsform beträgt dieser Zeitraum mindestens sechs Wochen.

### Kultivierungsbedingungen

Die Kultivierung wird bei einer für die Kultivierung der Stammzellen geeigneten Temperatur durchgeführt, bevorzugt bei 37°C.

Die Ausführung der Erfindung findet bevorzugt bei einer Sauerstoffkonzentration zwischen 1% und 25% statt, vorzugsweise bei einer Sauerstoffkonzentration von 21 %. In einer weiteren bevorzugten Ausführungsform liegt die Sauerstoffkonzentration zwischen 3% und 6%. In einer besonders bevorzugten Ausführungsform liegt die Sauerstoffkonzentration bei 4 %. Diese Sauerstoffpartialdrücke liegen in Bereichen (zwischen 3% und 6%), die in der Zellkultur, wie sie aus dem Stand der Technik bekannt ist, unüblich sind.

Die Kultivierung findet bei einer CO₂-Konzentration statt, die in einem Bereich liegt, der die Einstellung eines physiologischen pH-Werts im Kulturmedium erlaubt, bevorzugt bei 5% CO₂.

### Proliferation der Stammzellen

Das erfindungsgemäße Verfahren ist insbesondere geeignet, eine hohe Proliferationsrate der Zellen zu gewährleisten. Eine Ausführungsform der Erfindung ist daher das obengenannte Verfahren, gekennzeichnet dadurch, dass sich die Anzahl der Zellen pro Woche Kultivierung um mindestens 50% erhöht, bezogen auf die Zellzahl zu Beginn der Woche und während des ganzen Zeitraums der Kultivierung. Eine bevorzugte Ausführungsform der Erfindung ist daher das obengenannte Verfahren, gekennzeichnet dadurch, dass sich die Anzahl der Zellen pro Woche Kultivierung um mindestens 75% erhöht, bezogen auf die Zellzahl zu Beginn der Woche und während des ganzen Zeitraums der Kultivierung. Eine besonders bevorzugte Ausführungsform der Erfindung ist daher das obengenannte Verfahren, gekennzeichnet dadurch, dass sich die Anzahl der Zellen pro Woche Kultivierung um mindestens 100% erhöht, bezogen auf die Zellzahl zu Beginn der Woche und während des ganzen Zeitraums der Kultivierung. Eine besonders bevorzugte Ausführungsform der Erfindung ist daher das erfindungsgemäße Verfahren, gekennzeichnet dadurch, dass sich die Anzahl der Zellen pro Woche Kultivierung um mindestens 150% erhöht, bezogen auf die Zellzahl zu Beginn der Woche und während des ganzen Zeitraums der Kultivierung. Eine besonders bevorzugte Ausführungsform der Erfindung ist daher das obengenannte Verfahren, gekennzeichnet dadurch, dass sich die Anzahl der Zellen pro Woche Kultivierung um mindestens 200% erhöht, bezogen auf die Zellzahl zu Beginn der Woche und während des ganzen Zeitraums der Kultivierung. Mit dem erfindungsgemäßen Verfahren ist es möglich eine Proliferation zu erreichen, bei der sich die Anzahl der Zellen innerhalb von sechs Tage um 270 % erhöht, bezogen auf die Zellzahl zu Beginn der Woche. Dies bedeutet, dass sich die Zellzahl innerhalb einer Woche um über 300 % erhöht. Eine besonders bevorzugte Ausführungsform der Erfindung ist daher das obengenannte Verfahren, gekennzeichnet dadurch, dass sich die Anzahl der Zellen pro Woche Kultivierung um mindestens 300% erhöht, bezogen auf die Zellzahl zu Beginn der Woche und während des ganzen Zeitraums der Kultivierung. Insbesondere bevorzugte Ausführungsformen sind auch erfindungsgemäße Verfahren bei denen sich die Zellzahl um mehr als 300 % erhöht. Eine besonders bevorzugte Ausführungsform der Erfindung ist daher das obengenannte Verfahren, gekennzeichnet dadurch, dass sich die Anzahl der Zellen pro Woche Kultivierung um mindestens 400 %, 500 %, 600 %, 700 % oder 800 % erhöht, bezogen auf die Zellzahl zu Beginn der Woche und während des ganzen Zeitraums der Kultivierung.

Eine bevorzugte Ausführungsform der Erfindung ist daher das obengenannte Verfahren, gekennzeichnet dadurch, dass die Kultivierung mit einer Konzentration von unter 10⁶ Zellen pro Milliliter gestartet wird. Eine besonders bevorzugte Ausführungsform der Erfindung ist daher das obengenannte Verfahren, gekennzeichnet dadurch, dass die Kultivierung mit einer Konzentration von unter 10⁵ Zellen pro Milliliter gestartet wird. Eine besonders bevorzugte Ausführungsform der Erfindung ist daher das obengenannte Verfahren, gekennzeichnet dadurch, dass die Kultivierung mit einer Konzentration von unter 10⁴ Zellen pro Milliliter gestartet wird.

### Erhalt der Stammzell-/Pluripotenzeigenschaften der eingebrachten Stammzellen

In einer Ausführungsform der Erfindung besitzen noch mindestens 70 % der Zellen nach Beginn der Kultivierung Stammzelleigenschaften. In einer bevorzugten Ausführungsform der Erfindung besitzen noch mindestens 80 % der Zellen nach Beginn der Kultivierung Stammzelleigenschaften. In einer besonders bevorzugten Ausführungsform der Erfindung besitzen noch mindestens 90 % der Zellen nach Beginn der Kultivierung Stammzelleigenschaften. In einer besonders bevorzugten Ausführungsform der Erfindung besitzen noch mindestens 95 % der Zellen nach Beginn der Kultivierung Stammzelleigenschaften. Es ist in den nachfolgend aufgeführten Bespielen gezeigt, dass die Zellen nach Beginn der Kultivierung 97,7 % noch den Stammzellmarker Oct4 exprimieren. Daher besitzen in einer insbesondere bevorzugten Ausführungsform der Erfindung noch mindestens 97% der Zellen nach Beginn der Kultivierung Stammzelleigenschaften. In der am meisten bevorzugten Ausführungsform der Erfindung besitzen noch mindestens 99 % der Zellen nach Beginn der Kultivierung Stammzelleigenschaften. Für das Verfahren können sowohl embryonale als auch adulte Stammzellen eingesetzt werden. In beiden Fällen ermöglicht es die hohe Proliferationsrate, die Skalierbarkeit des Prozesses und einen Erhalt der Stammzelleigenschaften der eingebrachten Zellen.

Als Marker für den Erhalt der Stammzelleigenschaften dienen Stammzellmarker wie Oct4 (beschrieben in Pesce M, Scholer HR. Oct-4, Stem Cells 2001; 19:271-278), Tra-1-60 (beschrieben in Badcock, G., et al., 1999, Cancer Res. 59: 4715-4719.), Tra-1-81 (beschrieben in Henderson J.K. et. al. (2002). Stem Cells 20: 329-337), SSEA3, SSEA4 (beschrieben in Reubinoff B.E. et. al. (2000). Nature Biotechnology 18: 399-404), Nanog (Chambers I., et al., Cell. 2003 May 30;113(5):643-55.) und anderen Stammzellmarker (die vorgestellten Stammzellmarker werden auch diskutiert in: Brivanlou AH et al., Science. 2003 May 9;300(5621):913-6 und Henderson JK et al., Stem Cells. 2002;20(4):329-37). Deren Nachweis kann durch Antikörper gegen diese Stammzellmarker mittels Immunfluoreszenzmarkierung auf Objektträgern oder durch FACS-Analyse in Lösung erfolgen. Die Anzahl der angefärbten Zellen zeigt hierbei den Anteil der Stammzellen an. Weiterhin ist eine Quantifizierung der Expression der Stammzellmarker durch quantitative Reverse Transkriptase (RT) PCR, wie Echtzeit-RT-PCR, Kompetitions-PCR oder andere, möglich.

Der Anteil der Zellen mit Stammzelleigenschaften, die in die Kultur eingebracht werden, sollte mindestens 80% der kultivierten Zellen betragen. In einer bevorzugten Ausführungsform beträgt dieser Anteil 95%. In einer besonders bevorzugten Ausführungsform beträgt dieser Anteil 99%.

### Gefäße

Geeignete Gefäße für die Kultivierung der Stammzellen sind alle Gefäße, die es erlauben, das Medium zu bewegen und die Kulturbedingungen zu kontrollieren, wie Rollerflaschen, Spinnerflaschen, geregelte Bioreaktoren und andere geeignete Gefäße.

Rollerflaschen sind runde Zellkulturflaschen aus Plastik oder Glas mit einer Kulturfläche von zwischen 400 und 2000 cm². Die Zellen werden auf der gesamten inneren Oberfläche dieser Flaschen kultiviert, die Benetzung mit Medium wird dabei durch "Rollern", das heißt Drehen der Flaschen um die eigene Achse, gewährleistet.

Spinnerflaschen sind Glasgefäße (erhältlich z.B. von Bellco Glass Inc. oder Wheaton Scientific) mit Seitenarmen zur Medienzufuhr und zur Zell-Inokulation sowie einem Magnetrührer. Bevorzugterweise befindet sich im Boden der Flasche eine Erhebung, die eine Ansammlung der Carrier in der Mitte der Flasche verhindert.

Eine besondere Variante eines Spinners ist ein SuperSpinner. Ein SuperSpinner ist ein Spinner, der über einen Membranrührer begast wird. Dieser Membranrührer besteht aus einem Kunststoffhalter, auf den eine mikroporöse Begasungsmembran aus Polypropylen gewickelt ist. Durch diese Begasungsmembran erfolgt die Begasung des Mediums in einer für die Zellen besonders schonenden Weise.

Es ist auch möglich, in einem Spinner einen Paddelrührer zu verwenden. Dabei handelt es sich um einen Magnetrührer, an dem ein Teflonpaddel befestigt ist.

Es ist weiterhin möglich, einen Glasballrührer in einem Spinner zu verwenden. Er besteht aus einem Magneten, der in einen Glasball eingelassen ist.

Eine weitere Variante eines Rührers ist ein Scheibenrührer. Ein Scheibenrührer ist ein radial fördernder Rührer, während ein Propellerrührer eine axiale Strömung erzeugt. Auch ein Schaufelrührer und ein INTERMIG-Rührer fördern axial, ein Impeller-Rührer tangential bis radial.

Ein geregelter Bioreaktor ist ein Glas- oder Edelstahlgefäß, das mit einem Rührer und einem temperierbaren Mantel versehen ist. Mindestens ein System zur Regelung des Sauerstoffpartialdrucks ist vorhanden. Bevorzugterweise kann Medium zu- und abgeführt werden, eine Probenahme erfolgen und der pH-Wert eingestellt werden.

In einer besonders bevorzugten Ausführungsform besitzen die Bioreaktoren einen Begasungsring oder einen Begasungskorb um eine Begasung des Mediums zu ermöglichen.

Eine weitere Form eines Bioreaktors ist ein Airliftfermenter. Ein Airliftfermenter ist ein Bioreaktor, bei dem die Mischung nicht auf mechanischem Wege erfolgt. Statt dessen erfolgt der Energieeintrag in die Kultur über die Injektion komprimierter Luft vom Boden des Reaktors aus. Um eine effiziente Zirkulation zu gewährleisten, kann das System mit einem Leitrohr arbeiten (Schlaufenreaktor).

Zu den bevorzugten Ausführungsformen gehört der regelmäßige Austausch des Mediums. Eine besonders bevorzugte Ausführungsform ist der tägliche Wechsel des Mediums. Der Wechsel des Mediums kann kontinuierlich über geeignete Zellrückhaltesysteme erfolgen (siehe unten) oder diskontinuierlich durch unmittelbaren Austausch des gesamten Mediums. Der Wechsel des Mediums erfolgt dabei unter Bedingungen, welche die Sterilität im Inneren des Gefäßes erhalten. Bei Gefäßen, die klein genug sind, um in einer Sterilbank bearbeitet zu werden, kann ein Öffnen des Gefäßes und der Austausch des Mediums in einer Sterilbank erfolgen. Dabei kann nach Sedimentieren der Microcarrier das alte Medium abgesaugt und anschließend neues eingefüllt werden. Auch die Überführung der mit Stammzellen beladenen Microcarrier in ein frisches Gefäß mit frischem Medium ist eine bevorzugte Ausführungsform. Für Gefäße, die nicht in einer Sterilbank bearbeitbar sind, wird der Einsatz von Zellrückhaltesystemen, oder anderer Systeme, die einen sterilen Austausch von Medium gewährleisten, bevorzugt.

Einen kontinuierlichen Kultivierungsprozess gewährleisten in einer bevorzugten Ausführungsform Zellrückhaltesysteme. Diese ermöglichen einen Austausch des Mediums, wenn die Zellen samt Carriern im Reaktor verbleiben sollen. Erwünschte Ausführungsformen von Zellrückhaltesystemen in Verbindung mit der erfindungsgemäßen Methode sind Spinfilter und Steigrohre. Durch einen Spinfilter kann im Perfusionsmodus kontinuierlich Medium abgezogen werden. Durch den hydrostatischen Druck wird Flüssigphase vom Reaktor in das Innere des Spinfilters hineingedrückt. Ein durch den Deckel des Reaktors geführter Füllstandssensor registriert das Absinken des Kulturvolumens und frisches Medium wird nachgepumpt. Die Zellen werden durch den Filter zurückgehalten. Beim Steigrohr wird das Medium von oben abgezogen, nachdem die Zellen/Carrier sedimentiert sind.

Diese Gefäße und Systeme und andere zur Kultivierung von Zellen in Suspension geeignete Gefäße und Systeme werden in "Bioprozesstechnik" (H. Chmiel Hrsg., Gustav Fischer Verlag 1991) beschrieben.

Bevor eine Kultivierung mit Carriern stattfindet, können die Gefäße mit Dimethyldichlorosilan in einem organischen Lösemittel silanisiert werden, um ein Anheften der Zellen an der Gefäßoberfläche zu verhindern. Nach dem Trocknen der Silanisierungslösung werden die Gefäße mit destilliertem Wasser gespült.

### Legenden zu den Abbildungen

### Abbildung 1:

Wachstumskurve von menschlichen embryonalen Stammzellen (Zelllinie H9.2) auf unterschiedlichen Microcarriern. Die Zellen wurden in 20 ml Volumen in einem Spinner kultiviert und mit 30 upm mit einem Glasball-Rührer gerührt. Dabei wurde Intervallrühren mit einer Rührzeit von 30 Minuten und einer Ruhezeit von 30 Minuten eingesetzt. Das verwendete Medium war konditioniertes Medium von primären Fibroblasten der Maus. Es wurden Cytodex 3 (◆, Amersham) oder CultispherG (■, Perbio) als Microcarrier verwendet. Die Y-Achse bezeichnet die Gesamtzellzahl in der Kultur, die X-Achse den Zeitraum der Kultivierung in Stunden.

Die mittlere spezifische Wachstumsrate µ für Zellen auf dem Microcarrier Cytodex3 beträgt 0,014 h⁻¹ im Vergleich zu 0,013 h⁻¹ für nach dem Stand der Technik kultivierte Kontrollzellen.

### Abbildung 2:

Anfärbung von humanen embryonalen Stammzellen (Zelllinie H9.2, Amit et al (2000) Dev. Biol. 227: 271) aus Spinner (a Oct4-Färbung, b Kernfärbung (DAPI), c Overlay der beiden Färbungen) und zweidimensionaler Zellkultur (d Oct4-Färbung, e Kernfärbung (DAPI), f Overlay der beiden Färbungen) auf den Pluripotenzmarker Oct4. Die Zellen wurden für 240 Stunden im Spinner auf Cytodex3-Carriern kultiviert und mit 20 upm mit einem Glasball-Rührer gerührt. Anschließend wurden die Carrier auf Zellkulturschalen ausplattiert und nach weiteren 72 Stunden fixiert und auf den Pluripotenzmarker Oct4 gefärbt. Die Kontrollzellen wurden für 288 Stunden im zweidimensionalen System kultiviert.

### Abbildung 3:

Wachstumskurve von menschlichen embryonalen Stammzellen (Zellinie H9.2) auf Cytodex3-Mikrocarriern (Amersham). Die Zellen wurden in 50 ml Volumen in einem Spinner kultiviert und bei 20 upm mit einem Glasball-Rührer gerührt. Dabei wurde Intervallrühren mit einer Rührzeit von 30 Minuten und einer Ruhezeit von 30 Minuten (◆) bzw. Dauerrühren (■) eingesetzt. Das verwendete Medium war konditioniertes Medium von primären Fibroblasten der Maus. Die Y-Achse bezeichnet die Gesamtzellzahl in der Kultur, die X-Achse den Zeitraum der Kultivierung in Stunden.

Die mittlere spezifische Wachstumsrate µ für Zellen, die unter Dauerrühren kultiviert werden, beträgt-0,003 h⁻¹.

### Abbildung 4:

Menschliche embryonale Stammzellen (Zelllinie H9.2) wurden auf Cytodex3-Micorcarriern in 50 ml Volumen in einem Spinner kultiviert. Es wurde bei 20 upm mit einem Glasball-Rührer gerührt. Dabei wurde Intervallrühren mit einer Rührzeit von 30 Minuten und einer Ruhezeit von 30 Minuten bzw. Dauerrühren eingesetzt. Nach 15tägiger Kultivierung in konditioniertem Medium von primären Fibroblasten der Maus wurde eine quantitative Reverse Transkription-Polymerasekettenreaktion (qRT-PCR) für die Pluripotenzmarker Oct4, Nanog und Rex durchgeführt. Zur Berechnung der relativen Genexpression wurde die ΔΔCP-Methode verwendet, wobei der CP (Crossing Point)-Wert der Anzahl an PCR-Zyklen entspricht, die nötig ist, um ein konstant definiertes Fluoreszenzniveau zu erreichen. Am CP befindet sind in allen Reaktionsgefäßen die gleich Menge an neu synthetisierter DNA. Die Expression des jeweiligen Zielgens wird auf die Expression eines homogen exprimierten Referenzgens, eines sogenannten Houskeeping-Gens bezogen: ΔCP = CPZielgen - CPReferenzgen. Nach dieser Normierung wird vom ΔCP-Wert der Proben der ΔCP-Wert der Kontrolle abgezogen: ΔΔCP = ΔCPProbe - ΔCPKontrolle. Der relative Expressionsunterschied (Ration) einer Probe zur Kontrolle, normlaisiert auf ein Referenzgen, ergibt sich aus der Formel Ration =2^{-ΔΔCP}. Liegt der relative Expressionsunterschied unter 1, so ist das analysierte Gen in der Probe im Vergleich zu Kontrolle herunterreguliert, liegt er über 1, so ist das Gen im Vergleich zur Kontrolle heraufreguliert. Als Referenzgen wurde Gyceraldehyd-3-phosphat-Dehydrogenase (GAPDH) verwendet, als Kontrolle dienten Zellen der Linie H9.2, die unter konventionellen Bedingungen in adhärenter Kokultur mit Fibroblasten der Maus kultiviert wurden. Die Y-Achse bezeichnet den relativen Expressionsunterschied der auf der X-Achse dargestellten Pluripotenz-assoziierten Gene für unter Intervallrühren ( 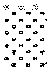 ) bzw. unter Dauerrühren ( 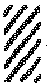 ) kultivierte embryonale Stammzellen.

### Abbildung 5:

Menschliche embryonale Stammzellen (Zelllinie H9.2) wurden für 14 Tage auf Cytodex3-Carriern in einem Spinner mit Glasballrührer kultiviert. Es wurde Intervallrühren bei 20 upm mit einer Rührzeit von 30 Minuten und einer Ruhezeit von 30 Minuten eingesetzt. Um nachzuweisen, dass die so kultivierten embryonalen Stammzellen noch pluripotent, also in der Lage sind, durch Differenzeirung Zellen aller drei Keimblätter zu bilden, wurde nach 14tägiger Kultivierung eine spontane Embryoidkörper-Bildung der embryonalen Stammzellen in nicht-konditioniertem, serumhaltigen Medium induziert. Nach drei Wochen wurden die Embryoidkörper fixiert, geschnitten und auf die keimblattspezifischen Marker Alpha-Fetoprotein (Endoderm), Desmin (Mesoderm) und Cytokeratin (Ektoderm) gefärbt. a Alpha-Fetoprotein-Färbung, b Kernfärbung (DAPI), c Overlay der Färbungen a und b; d Desmin-Färbung, e Kerfärbung (DAPI), f Overlay der Färbungen d und e; g Cytokeratin-Färbung, h Kernfärbung (DAPI) i Overlay der Färbungen g und h

### Referenzbeispiele

### Referenzbeispiel 1:

5,5 x 10⁵ humane embryonale Stammzellen (Zelllinie H9.2, Amit et al (2000) Dev. Biol. 227: 271) pro ml werden zusammen mit 1,5 mg/ml Cytodex 3 (Amersham) oder 0,5 mg/ml CultispherG (Perbio) Microcarriern in einen Spinner (z.B. Bellco) eingebracht. Als Kulturmedium wird Knockout-DMEM mit 20% Serum Replacement, 1% nicht essentielle Aminosäuren, 0,5% L-Glutamin und 0,1mM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen liegt bei 10 ml. Nach anfänglicher Ruhephase von einem Tag wird das Mediumvolumen auf 20 ml erhöht und mit Intervallrühren begonnen. Dabei wurde ein Glasballrührer verwendet, und eine Rührerdrehzahl von 30 upm eingestellt. Intervallrühren mit einer Rührzeit von 30 Minuten und einer Ruhezeit von 30 Minuten wird eingesetzt. Der Sauerstoffpartialdruck liegt bei 21%. Der pH-Wert wird auf einem physiologischen Wert zwischen 7,2 und 7,4 gehalten. Das Medium wird täglich komplett ausgetauscht.

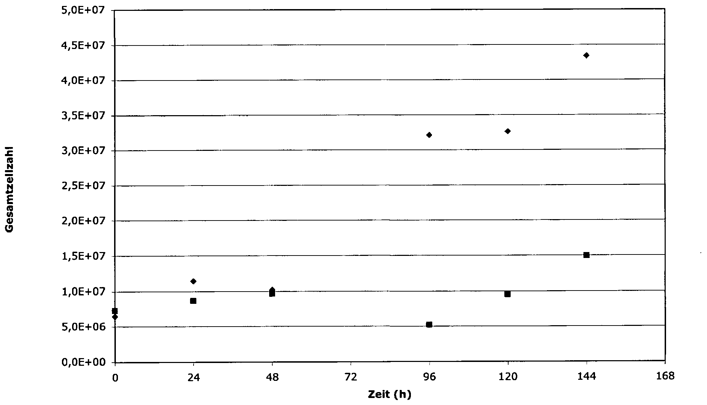

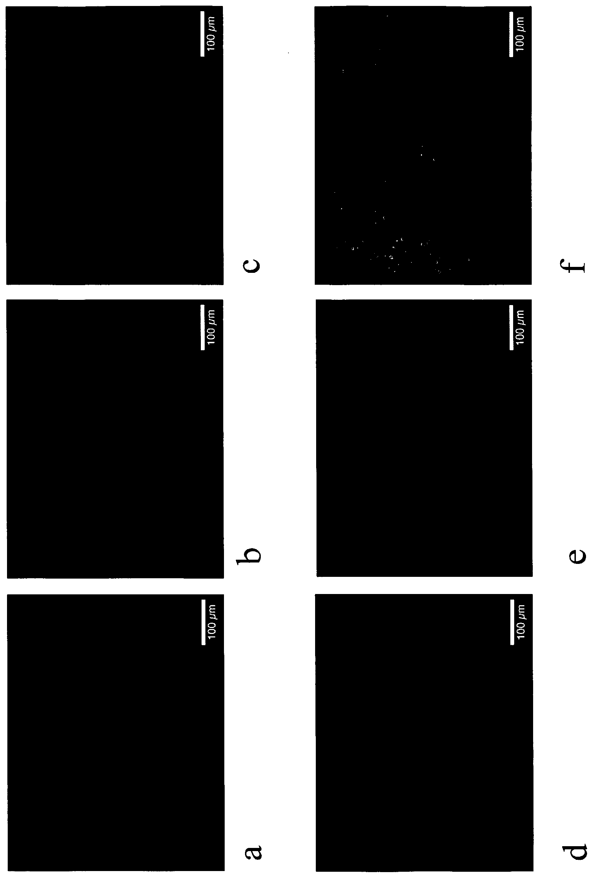

Nach zehntägiger Kultivierung wurden die Zellen entnommen, die Zellen plattiert und bei den mit Cytodex3 inkubierten Microcarriern eine Oct4-Färbung durchgeführt. In der Oct4-Färbung sind 97,7% der Zellen in den nach Plattieren aus den Carrier-Zell-Aggregaten hervorgegangenen Kolonien positiv für den Pluripotenzmarker (siehe Abbildung 2). Somit ist gezeigt, dass erfindungsgemäßes Intervallrühren bei der Kultivierung von Stammzellen maßgeblich zum Erhalt der Stammzelleigenschaften kultivierter Stammzellen beiträgt und einen deutlichen Vorteil über die Kultivierung in zweidimensionaler Kultur darstellt. Nach sechstägiger Kultivierung hat sich die Zellzahl im Vergleich zur Ausgangszellzahl um 780 % (Cytodex3) bzw. 270 % (CultisphereG) erhöht (siehe Abbildung 1).

### Referenzbeispiel 2:

3,5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen geregelten Bioreaktor (z.B. B. Braun) eingebracht. Als Kulturmedium wird Knockout-DMEM mit 20% Serum Replacement, 1% nicht essentielle Aminosäuren, 0,5% L-Glutamin und 0,1mM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen beträgt 51. Nach anfänglicher Ruhephase von einem Tag wird mit dem Rühren begonnen. Eine Rührerdrehzahl von 30 upm ist eingestellt. Der Sauerstoffpartialdruck im Reaktor beträgt 5%. Der pH-Wert wird auf einem physiologischen Wert zwischen 7,2 und 7,4 gehalten. Das Medium wird täglich komplett ausgetauscht. Nach Kultivierung für 10 Tage werden die Zellen aus dem Gefäß entnommen.

### Referenzbeispiel 3:

3, 5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner inokuliert. Als Kulturmedium wird Knockout-DMEM mit 20% Serum Replacement, 1% nicht essentielle Aminosäuren, 0,5% L-Glutamin und 0,1mM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen liegt bei 20 ml. Nach anfänglicher Ruhephase von einem Tag wird mit dem Intervallrühren begonnen. Ein Paddelrührer wird verwendet, dabei wird eine Rührerdrehzahl von 20 upm eingestellt. Intervallrühren mit einer Rührzeit von 30 Minuten und einer Ruhezeit von 30 Minuten wird eingesetzt. Der Sauerstoffpartialdruck liegt bei 21%. Der pH-Wert wird auf einem physiologischen Wert zwischen 7,2 und 7,4 gehalten. Das Medium wird täglich komplett ausgetauscht. Nach Kultivierung für 10 Tage werden die Zellen aus dem Gefäß entnommen.

### Referenzbeispiel 4:

3,5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner inokuliert. Als Kulturmedium wird Knockout-DMEM mit 20% Serum Replacement, 1% nicht essentielle Aminosäuren, 0,5% L-Glutamin und 0,1mM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen liegt bei 20 ml. Nach anfänglicher Ruhephase von einem Tag wird mit dem Intervallrühren begonnen. Ein Glasballrührer wird verwendet, dabei wird eine Rührerdrehzahl von 20 upm eingestellt. Der Sauerstoffpartialdruck liegt bei 21%. Der pH-Wert wird auf einem physiologischen Wert zwischen 7,2 und 7,4 gehalten. Das Medium wird täglich komplett ausgetauscht. Nach Kultivierung für 15 Tage werden die Zellen aus dem Gefäß entnommen.

### Referenzbeispiel 5:

3,5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner inokuliert. Als Kulturmedium wird Knockout-DMEM mit 20% FCS (Hyclone), 1% nicht essentielle Aminosäuren, 0,5% L-Glutamin, 0,1mM 2-Mercaptoethanol und 4ng/ml bFGF (alle Invitrogen) verwendet.

Das Kulturvolumen liegt bei 20 ml. Nach anfänglicher Ruhephase von einem Tag wird mit dem Intervallrühren begonnen. Ein Paddelrührer wird verwendet, dabei wird eine Rührerdrehzahl von 20 upm eingestellt. Intervallrühren mit einer Rührzeit von 30 Minuten und einer Ruhezeit von 30 Minuten wird eingesetzt. Der Sauerstoffpartialdruck liegt bei 21%. Der pH-Wert wird auf einem physiologischen Wert zwischen 7,2 und 7,4 gehalten. Das Medium wird täglich komplett ausgetauscht. Nach Kultivierung für 20 Tage werden die Zellen aus dem Gefäß entnommen.

### Referenzbeispiel 6:

4 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen geregelten Bioreaktor eingebracht. Als Kulturmedium wird Knockout-DMEM mit 15% Serum Replacement, 1% nicht essentielle Aminosäuren, 2mM L-Glutamin, 0,1mM 2-Mercaptoethanol 4ng/ml bFGF (alle Invitrogen), 1000U/ml LIF (Chemicon) und 0,12ng/ml TGFβ1 (R&D Systems) verwendet.

Die Microcarrier werden vor dem Einbringen in das Gefäß zusammen mit den Microcarriern in einer Zellkulturflasche in einem Volumen von 50 ml inkubiert, um ein Anheften der Zellen zu ermöglichen. Danach werden die mit Zellen beladenen Microcarrier in einen Spinner mit einem Kulturvolumen von 500 ml eingebracht. Ein Paddelrührer wird verwendet, dabei wird eine Rührerdrehzahl von 20 upm eingestellt. Intervallrühren mit einer Rührzeit von 30 Minuten und einer Ruhezeit von 30 Minuten wird eingesetzt. Der Sauerstoffpartialdruck liegt bei 21%. Der pH-Wert wird auf einem physiologischen Wert zwischen 7,2 und 7,4 gehalten. Das Medium wird täglich komplett ausgetauscht. Im Abstand von drei Tagen werden Microcarrier aus dem Spinner steril entnommen, um eine Kontrolle der Stammzelleigenschaften durchzuführen. Entnommenes Medium wird durch frisches ersetzt. Nach Kultivierung für 15 Tage werden die Zellen aus dem Gefäß entnommen.

### Referenzbeispiel 7:

3 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner eingebracht. Als Kulturmedium wird ExVivo (Cambrex) mit 40ng/ml bFGF (Invitrogen) und 15 ng/ml flt3-Ligand (Peprotech) verwendet.

Das Kulturvolumen liegt bei 20 ml. Nach anfänglicher Ruhephase von zwölf Stunden wird mit dem Intervallrühren begonnen. Ein Paddelrührer wird verwendet, dabei wird eine Rührerdrehzahl von 20 upm eingestellt. Intervallrühren mit einer Rührzeit von 30 Minuten und einer Ruhezeit von 30 Minuten wird eingesetzt. Der Sauerstoffpartialdruck liegt bei 21%. Der pH-Wert wird auf einem physiologischen Wert zwischen 7,2 und 7,4 gehalten. Das Medium wird täglich komplett ausgetauscht. Im Abstand von einer Woche wird die Hälfte des Mediums mit den Microcarriern entnommen und durch frisches Medium und frische Microcarrier ersetzt. Nach Kultivierung für 21 Tage werden die Zellen aus dem Gefäß entnommen.

### Referenzbeispiel 8:

3 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner eingebracht. Als Kulturmedium wird Knockout-DMEM mit 20% Serum Replacement, 1% nicht essentielle Aminosäuren, 0,5% L-Glutamin und 0,1mM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen liegt bei 20 ml. Nach anfänglicher Ruhephase von einem Tag wird mit Intervallrühren begonnen. Ein Glasballrührer wird verwendet, dabei wird eine Rührerdrehzahl von 30 upm eingestellt. Intervallrühren mit einer Rührzeit von 30 Minuten und einer Ruhezeit von 30 Minuten wird eingesetzt. Der Sauerstoffpartialdruck liegt bei 21%. Der pH-Wert wird auf einem physiologischen Wert zwischen 7,2 und 7,4 gehalten. Das Medium wird täglich komplett ausgetauscht.

Nach 14 Tagen wird das Medienvolumen auf 40 ml erhöht, Die Carrierkonzentration wird bei 1,5 mg/ml für den Carrier Cytodex 3 oder bei 0,5 mg/ml für den Carrier CultispherG gehalten. Das Medium wird täglich komplett ausgetauscht. Nach Kultivierung für insgesamt 4 Wochen werden die Zellen aus dem Gefäß entnommen.

### Referenzbeispiel 9

3,5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner (z.B. B. Braun) eingebracht. Als Kulturmedium wird Knockout-DMEM mit 20% Serum Replacement, 1% nicht essentielle Aminosäuren, 0,5% L-Glutamin und 0,1mM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen liegt bei 20 ml. Nach anfänglicher Ruhephase von zwölf Studen wird mit dem Intervallrühren begonnen. Ein Glasballrührer wird verwendet, dabei wird eine Rührerdrehzahl von 30 upm eingestellt. Intervallrühren mit einer Rührzeit von 30 Minuten und einer Ruhezeit von 30 Minuten wird eingesetzt. Der Sauerstoffpartialdruck liegt bei 4%. Der pH-Wert wird auf einem physiologischen Wert zwischen 7,2 und 7,4 gehalten. Das Medium wird täglich komplett ausgetauscht. Nach Kultivierung für insgesamt 6 Wochen werden die Zellen aus dem Gefäß entnommen.

### Referenzbeispiel 10 (Dauerrühren)

3,5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner (z.B. Bellco) eingebracht. Als Kulturmedium wird Knockout-DMEM mit 20% Serum Replacement, 1% nicht essentielle Aminosäuren, 0,5% L-Glutamin und 0,1mM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen liegt bei 20 ml. Nach anfänglicher Ruhephase von zwölf Stunden wird mit dem Rühren begonnen. Ein Glasballrührer wird verwendet, dabei wird eine Rührerdrehzahl von 20 upm eingestellt. Das Medium wird täglich komplett ausgetauscht. Nach 15tägiger Kultivierung werden die Zellen aus dem Gefäß entnommen.

### Referenzbeispiel 11 (normoxisch, Reaktor)

3,5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen geregelten Bioreaktor (z.B. B. Braun) eingebracht. Als Kulturmedium wird Knockout-DMEM mit 20% Serum Replacement, 1% nicht essentielle Aminosäuren, 0,5% L-Glutamin und 0,ImM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen beträgt 5 1. Nach anfänglicher Ruhephase von einem Tag wird mit dem Rühren begonnen. Eine Rührerdrehzahl von 30 upm ist eingestellt. Der Sauerstoffpartialdruck im Reaktor beträgt 21%. Der pH- Wert wird auf einem physiologischen Wert zwischen 7,2 und 7,4 gehalten. Das Medium wird täglich komplett ausgetauscht. Nach Kultivierung für 10 Tage werden die Zellen aus dem Gefäß entnommen.

### Referenzbeispiel 12 (andere Medienzusammensetzung)

4 x 10⁵ humane embryonale Stammzellen (Zelllinie H9.2) pro ml werden zusammen mit 1,5 mg/ml Cytodex 3 (Amersham) Microcarriern in einen Spinner (z.B. Bellco) eingebracht. Als Kulturmedium wird Knockout-DMEM mit 20% Serum Replacement, 1% nicht essentielle Aminosäuren, 2mM L-Glutamin, 0,ImM 2-Mercaptoethanol 40ng/ml bFGF (alle Invitrogen), 0,5µg/ml murines Noggin (R&D Systems) verwendet.

Nach eintägiger statischer Kultivierung in 25 ml Volumen wird das Kulturvolumen auf 50 ml eingestellt. Ein Paddelrührer wird bei einer Rührerdrehzahl von 30 upm verwendet. Intervallrühren mit einer Rührzeit von 30 Minuten und einer Ruhezeit von 30 Minuten wird eingesetzt. Das Medium wird täglich komplett ausgetauscht. Nach Kultivierung für 10 Tage werden die Zellen aus dem Gefäß entnommen.

### Referenzbeispiel 13

3,5 x 10⁵ humane embryonale Stammzellen (Zelllinie H9.2) pro ml werden zusammen mit 1,5 mg/ml Cytodex 3 (Amersham) oder 0,5 mg/ml CultispherG (Perbio) Microcarriern in einen Spinner (z.B. B. Braun) eingebracht. Als Kulturmedium wird Knockout-DMEM mit 20% Serum Replacement, 1% nicht essentielle Aminosäuren, 0,5% L-Glutamin und 0,ImM 2-Mercaptoethanol (alle Invitrogen) verwendet, das für 24 Stunden von gamma-bestrahlten primären Maus-Fibroblasten (zur Präparation der Fibroblasten: Siemen, H., Diplomarbeit 2003, Institut für Anatomie, Universität Regensburg) aus dem Mausstamm CD1 konditioniert wurde. 140000 Fibroblasten pro ml zu konditionierendes Medium werden verwendet. Vor dem Einsatz für die hESC-Kultur wird das konditionierte Medium gefiltert und 4 ng/ml bFGF (Invitrogen) werden zugegeben.

Das Kulturvolumen liegt bei 50 ml. Nach anfänglicher Ruhephase von zwölf Stunden wird mit dem Intervallrühren begonnen. Ein Glasballrührer wird verwendet, dabei wird eine Rührerdrehzahl von 20 upm eingestellt. Intervallrühren mit einer Rührzeit von 30 Minuten und einer Ruhezeit von 30 Minuten wird eingesetzt. Der Sauerstofrpartialdruck liegt bei 21%. Der pH- Wert wird auf einem physiologischen Wert zwischen 7,2 und 7,4 gehalten. Das Medium wird täglich komplett ausgetauscht.

Als Kontrolle diente eine Spinner-Kultur, bei der - bei ansonsten gleichen Bedingungen - Dauerrühren statt Intervallrühren eingesetzt wurde.

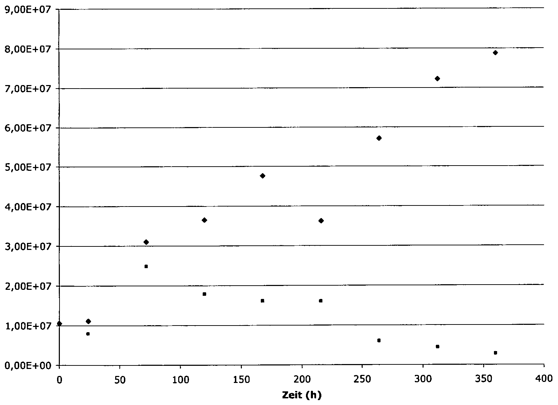

Wie aus Abbildung 3 hervorgeht, ist die Proliferation von Stammzellen, die unter erfindungsgemäßem Intervallrühren kultiviert werden, deutlich besser als die von Stammzellen, bei denen Dauerrühren zum Einsatz kommt. Unter Intervallrühren lässt sich eine Zunahme der Zellzahl beobachten. Hingegen ist unter Dauerrühren sogar eine Abnahme der Zellzahl zu beobachten.

### Referenzbeispiel 14

Es wurde unter den gleichen Kulturbedingungen wie in Referenzbeispiel 13 der Erhalt der Pluripotenzeigenschaften bzw. Stammzelleigenschaften unter Intervallrühren bzw. als Kontrolle Dauerrühren gemessen. Nach 15tägiger Kultivierung in konditioniertem Medium von primären Fibroblasten der Maus wurde eine quantitative Reverse Transkription-Polymerasekettenreaktion (qRT-PCR) für die Pluripotenzmarker Oct4, Nanog und Rex durchgeführt. Zur Berechnung der relativen Genexpressionen wurde die ΔΔCP-Methode verwendet, wobei der CP (Crossing Point)- Wert der Anzahl an PCR-Zyklen entspricht, die nötig ist, um ein konstant definiertes Fluoreszenzniveau zu erreichen. Am CP befindet sich in allen Reaktionsgefäßen die gleiche Menge an neu synthetisierter DNA. Die Expression des jeweiligen Zielgens wird auf die Expression eines homogen exprimierten Referenzgens, eines sogenannten Housekeeping-Gens bezogen: ΔCP = CPZielgen - CPReferenzgen. Nach dieser Normierung wird vom ΔCP-Wert der Proben der ΔCP-Wert der Kontrolle abgezogen: ΔΔCP = ΔCPProbe - ΔCPKontrolle. Der relative Expressionsunterschied (Ratio) einer Probe zur Kontrolle, normalisiert auf ein Referenzgen, ergibt sich aus der Formel Ratio = 2^{-ΔΔCP}. Liegt der relative Expressionsunterschied unter 1, so ist das analysierte Gen in der Probe im Vergleich zur Kontrolle herunterreguliert, liegt er über 1, so ist das Gen im Vergleich zur Kontrolle heraufreguliert. Als Referenzgen wurde Glyceraldehyd-3-phosphat-Dehydrogenase (GAPDH) verwendet, als Kontrolle dienten Zellen der Linie H9.2, die unter konventionellen Bedingungen in adhärenter Kokultur mit Fibroblasten der Maus kultiviert wurden.

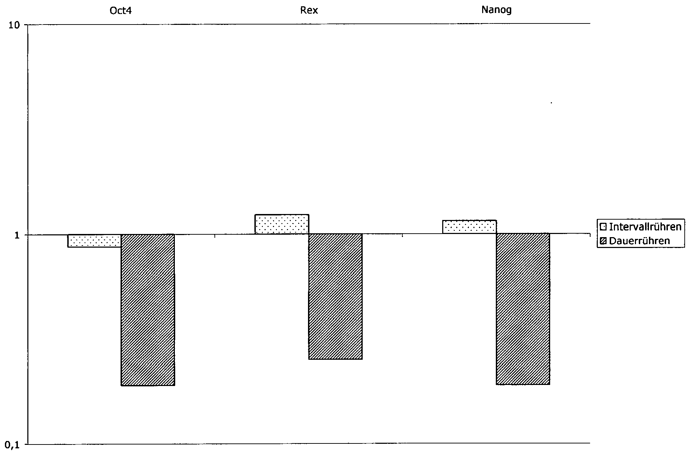

Wie deutlich aus Abbildung 4 hervorgeht, verlieren unter Dauerrühren die ES-Zellen die Zellmarker für Pluripotenz/ES-Zellen Eigenschaften. Dies zeigt an, dass ES-Zellen unter Dauerrühren ihre Pluripotenz/ES-Zellen Eigenschaften verlieren. Hingegen geht aus Abbildung 4 ebenfalls hervor, dass das erfindungsgemäße Intervallrühren den Erhalt der Pluripotenz/ES-Zellen Eigenschaften ermöglicht. Somit ist auf eindrucksvolle Weise die Vorteilhaftigkeit des Intervallrührens gegenüber dem Dauerrühren gezeigt.

### Referenzbeispiel 15

Im folgenden wird gezeigt, dass die unter Intervallrühren kultivierten Zellen die Fähigkeit besitzen zu Zellen aller drei Keimblätter zu differenzieren. Die Zellen wurden unter den gleichen Kulturbedingungen wie in Referenzbeispiel 14 unter Intervallrühren kultiviert. Um nachzuweisen, dass die so kultivierten embryonalen Stammzellen noch pluripotent, also in der Lage sind, durch Differenzierung Zellen aller drei Keimblätter zu bilden, wurde nach 14tägiger Kultivierung eine spontane Embryoidkörper-Bildung der embryonalen Stammzellen in nicht-konditioniertem, serumhaltigen Medium induziert. Nach drei Wochen wurden die Embryoidkörper fixiert, geschnitten und auf die keimblattspezifischen Marker Alpha-Fetoprotein (Endoderm), Desmin (Mesodeπn) und Cytokeratin (Ektodeπn) gefärbt.

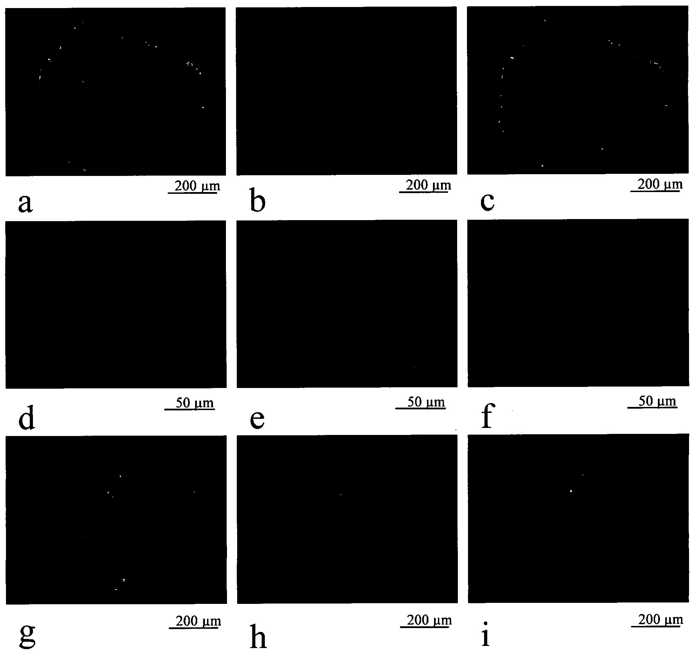

Abbildung 5 demonstriert, dass die unter erfindungsgemäßen Intervallrühren kultivierten ES-Zellen in der Lage sind, in die Zellen aller drei Keimblätter zu differenzieren. ES-Zellen, die unter erfindungsgemäßes Intervallrühren kultiviert wurden, sind demnach nicht nur in der Lage ohne Verlust ihrer Pluripotenz/ES-Zellen Eigenschaften zu proliferieren, sondern besitzen die Fähigkeit nach gezielter Induktion zu Zelltypen aller drei Keimblätter differenzieren.

## Patentansprüche

1. Ein Verfahren zur Vermehrung von Stammzellen, umfassend:
das Einbringen von Medium, Microcarriern und Stammzellen in ein Gefäß,
die Kultivierung von Stammzellen auf Microcarriern in diesem Gefäß, wobei die Zellen nach Beginn der Kultivierung ihre Stammzelleigenschaften nicht verlieren,
und
die Entnahme von Zellen auf Microcarriern aus dem Gefäß;
wobei nach Beginn der Kultivierung das Medium abwechselnd für zwischen 10 und 100 Minuten bewegt wird und zwischen 10 und 60 Minuten nicht bewegt wird.

2. Das Verfahren gemäß Anspruch 1, wobei die Stammzellen vor dem Einbringen in das Gefäß mit den Microcarriern inkubiert werden, um den Stammzellen ein Anheften an die Microcarrier zu ermöglichen.

3. Das Verfahren gemäß Anspruch 1, wobei nach dem Einbringen von Medium und Microcarriern in das Gefäß die Stammzellen in das Gefäß eingebracht werden.

4. Das Verfahren gemäß Anspruch 3, wobei, nachdem die Stammzellen in das Gefäß eingebracht wurden, eine Ruhephase stattfindet, **dadurch gekennzeichnet, dass** das Medium im Gefäß für einen Zeitraum von maximal einem Tag nicht bewegt wird, um eine Anheftung der Zellen an die Microcarrier zu ermöglichen.

5. Das Verfahren gemäß Anspruch 1, wobei die Stammzellen embryonale Stammzellen sind.

6. Das Verfahren gemäß Anspruch 1, wobei die Stammzellen adulte Stammzellen sind.

7. Das Verfahren gemäß Anspruch 1, wobei das Medium abwechselnd für zwischen 15 und 45 Minuten bewegt wird und zwischen 15 und 45 Minuten nicht bewegt wird.

8. Das Verfahren gemäß Anspruch 1, wobei dieses Gefäß eine Spinnerflasche ist.

9. Das Verfahren gemäß Anspruch 1, wobei dieses Gefäß ein geregelter Bioreaktor ist.

10. Das Verfahren gemäß Anspruch 8 oder 9, wobei in diesem Gefäß das Bewegen des Mediums durch Rühren mit einem Rührer bewirkt wird.

11. Das Verfahren gemäß Anspruch 1, **dadurch gekennzeichnet, dass** das Medium konditioniert wird.

12. Das Verfahren gemäß Anspruch 11, wobei das Medium dadurch konditioniert wird, dass in dem Medium zwischen 1 und 3 Tagen Zellen kultiviert wurden, die keine Stammzellen sind.

13. Das Verfahren gemäß Anspruch 1, wobei das Medium dadurch konditioniert wird, dass in dem Medium für einen Tag Zellen kultiviert wurden, die keine Stammzellen sind.

14. Das Verfahren gemäß Anspruch 12 oder 13, wobei die Zellen, die keine Stammzellen sind, vor dem Einbringen der Stammzellen aus dem Medium entfernt werden.

15. Das Verfahren gemäß Anspruch 12 oder 13, wobei die Zellen, die keine Stammzellen sind, Fibroblasten sind.

16. Das Verfahren gemäß Anspruch 1, wobei die Microcarrier aus einem Trägermaterial und einer Beschichtung bestehen.

17. Das Verfahren gemäß Anspruch 1, wobei die Microcarrier aus einem einheitlichen Material bestehen.

18. Das Verfahren gemäß Anspruch 1, wobei die Kultivierung mindestens zwei Wochen andauert.

19. Das Verfahren gemäß Anspruch 1, wobei die Kultivierung ohne fötales Kälberserum erfolgt.

20. Das Verfahren gemäß Anspruch 1, wobei die Kultivierung mit Serumersatz erfolgt.

21. Das Verfahren gemäß Anspruch 1, wobei die Kultivierung in Gegenwart von basic fibroblast growth factor (bFGF) erfolgt.

22. Das Verfahren gemäß Anspruch 1, wobei die Entnahme von Zellen aus dem Gefäß **dadurch gekennzeichnet ist, dass** nur ein Teil des Mediums, das die mit Zellen besetzten Microcarrier enthält, entnommen wird, und dieser Teil des Mediums durch Einbringen von frischem Medium und neue, nicht mit Zellen besetzte Microcarrier ersetzt wird.

23. Das Verfahren gemäß Anspruch 1, wobei mindestens 99% der Zellen nach Beginn der Kultivierung Stammzelleneigenschaften besitzen.

24. Das Verfahren gemäß Anspruch 1, wobei sich die Anzahl der Zellen innerhalb von einer Woche Kultivierung mindestens um 50% bezogen auf die Ausgangszellzahl erhöht.

25. Das Verfahren gemäß Anspruch 1, wobei sich die Anzahl der Zellen innerhalb von einer Woche Kultivierung mindestens um 600% bezogen auf die Ausgangszellzahl erhöht.

26. Das Verfahren gemäß Anspruch 1, wobei die Kultivierung der Zellen mit einer Konzentration von unter 10⁶ Zellen pro Milliliter gestartet wird.

27. Das Verfahren gemäß Anspruch 1, wobei nach der Entnahme der Zellen mit den Microcarriern die Zellen von den Microcarriern abgelöst werden.

28. Das Verfahren gemäß Anspruch 1, wobei die Entnahme von Zellen zum Zweck der Überprüfung des Erhaltes der Stammzelleigenschaften vorgenommen wird.

## Claims

1. A method of proliferating stem cells comprising:
introducing medium, microcarriers and stem cells into a vessel,
cultivating stem cells on microcarriers in said vessel, wherein the cells do not lose their stem cell properties after the beginning of cultivating,
and
harvesting cells on microcarriers from said vessel;
wherein after the beginning of cultivating the medium is alternately agitated for between 10 and 100 minutes and not agitated between 10 and 60 minutes.

2. The method of claim 1, wherein the stem cells are incubated with the microcarriers prior to introduction into the vessel to allow the stem cells to attach to the microcarriers.

3. The method of claim 1, wherein the stem cells are introduced into the vessel after the introduction of medium and microcarriers into the vessel.

4. The method of claim 3, wherein a resting phase occurs after the stem cells have been introduced into the vessel, **characterized in that** the medium in the vessel is not agitated for a maximum of one day to allow attachment of the cells to the microcarriers.

5. The method of claim 1, wherein the stem cells are embryonic stem cells.

6. The method of claim 1, wherein the stem cells are adult stem cells.

7. The method of claim 1, wherein the medium is alternately agitated for between 15 and 45 minutes and not agitated between 15 and 45 minutes.

8. The method of claim 1, wherein said vessel is a spinner flask.

9. The method of claim 1, wherein said vessel is a controlled bioreactor.

10. The method of claim 8 or 9, wherein the agitation of the medium in said vessel is effected by stirring with a stirrer.

11. The method of claim 1, **characterized in that** the medium is conditioned.

12. The method of claim 11, wherein the medium is conditioned by culturing cells that are not stem cells in the medium for between 1 to 3 days.

13. The method of claim 1, wherein the medium is conditioned by culturing cells that are not stem cells in the medium for one.

14. The method of claim 12 or 13, wherein the cells which are not stem cells are removed from the medium prior to introduction of the stem cells.

15. The method of claim 12 or 13, wherein the cells which are not stem cells are fibroblasts.

16. The method of claim 1, wherein the microcarriers consist of a carrier material and a coating.

17. The method of claim 1, wherein the microcarriers consist of a uniform material.

18. The method of claim 1, wherein culturing lasts at least two weeks.

19. The method of claim 1, wherein culturing is carried out without fetal calf serum.

20. The method of claim 1, wherein culturing is carried out with serum replacement.

21. The method of claim 1, wherein culturing is carried out in the presence of basic fibroblast growth factor (bFGF).

22. The method of claim 1, wherein harvesting the cells from the vessel is **characterized in that** only part of the medium containing the cell-occupied microcarriers is harvested and said part of the medium is replaced by introducing fresh medium and new, non-cell-occupied microcarriers.

23. The method of claim 1, wherein at least 99% of the cells have stem cell properties after initiating culturing.

24. The method of claim 1, wherein the number of cells increases within a week of culturing at least by 50% based on the starting cell number.

25. The method of claim 1, wherein the number of cells increases within a week of culturing at least by 600% based on the starting cell number.

26. The method of claim 1, wherein culturing of the cells is started at a concentration of below 10⁶ cells per milliliter.

27. The method of claim 1, wherein after harvesting the cells with the microcarriers the cells are detached from the microcarriers.

28. The method of claim 1, wherein harvesting the cells is performed for the purpose of verifying the maintenance of stem cell properties.

## Revendications

1. Procédé permettant de multiplier les cellules souches, comprenant :
l'introduction du milieu, des microsupports et des cellules souches dans un récipient,
la culture des cellules souches sur des microsupports dans ledit récipient, dans lequel les cellules ne perdent pas leurs propriétés de cellules souches après le début de la culture,
et
le prélèvement des cellules sur les microsupports hors du récipient ;
dans lequel le milieu est tour à tour agité pendant entre 10 et 100 minutes et non agité pendant entre 10 et 60 minutes après le début de la culture.

2. Procédé selon la revendication 1, dans lequel les cellules souches sont incubées avec les microsupports avant l'introduction dans le récipient afin de permettre aux cellules souches d'adhérer sur les microsupports.

3. Procédé selon la revendication 1, dans lequel les cellules souches sont introduites dans le récipient après l'introduction du milieu et des microsupports dans le récipient.

4. Procédé selon la revendication 3, dans lequel, une fois que les cellules souches ont été introduites dans le récipient, une phase de pause intervient, **caractérisé en ce que** le milieu dans le récipient n'est pas agité pendant une durée d'au maximum une journée afin de permettre une adhésion des cellules sur les microsupports.

5. Procédé selon la revendication 1, dans lequel les cellules souches sont des cellules souches embryonnaires.

6. Procédé selon la revendication 1, dans lequel les cellules souches sont des cellules souches adultes.

7. Procédé selon la revendication 1, dans lequel le milieu est tour à tour agité pendant entre 15 et 45 minutes et non agité pendant entre 15 et 45 minutes.

8. Procédé selon la revendication 1, dans lequel ledit récipient est une fiole rotative.

9. Procédé selon la revendication 1, dans lequel ledit récipient est un bioréacteur régulé.

10. Procédé selon la revendication 8 ou 9, dans lequel l'agitation du milieu dans ledit récipient est réalisée en remuant avec un agitateur.

11. Procédé selon la revendication 1, **caractérisé en ce que** le milieu est conditionné.

12. Procédé selon la revendication 11, dans lequel le milieu est conditionné du fait que des cellules qui ne sont pas des cellules souches ont été cultivées dans le milieu pendant entre 1 et 3 jours.

13. Procédé selon la revendication 1, dans lequel le milieu est conditionné du fait que des cellules qui ne sont pas des cellules souches ont été cultivées dans le milieu pendant une journée.

14. Procédé selon la revendication 12 ou 13, dans lequel les cellules qui ne sont pas des cellules souches sont éliminées du milieu avant l'introduction des cellules souches.

15. Procédé selon la revendication 12 ou 13, dans lequel les cellules qui ne sont pas des cellules souches sont des fibroblastes.

16. Procédé selon la revendication 1, dans lequel les microsupports se composent d'un matériau support et d'un revêtement.

17. Procédé selon la revendication 1, dans lequel les microsupports se composent d'un matériau uniforme.

18. Procédé selon la revendication 1, dans lequel la culture dure au moins deux semaines.

19. Procédé selon la revendication 1, dans lequel la culture s'effectue sans sérum foetal de veau.

20. Procédé selon la revendication 1, dans lequel la culture s'effectue avec un substitut de sérum.

21. Procédé selon la revendication 1, dans lequel la culture s'effectue en présence du facteur de croissance des fibroblastes basiques (bFGF).

22. Procédé selon la revendication 1, dans lequel le prélèvement des cellules hors du récipient est **caractérisé en ce que** seule une partie du milieu qui contient les microsupports pourvus des cellules est prélevée et que cette partie du milieu est remplacée par l'introduction de milieu frais et de nouveaux microsupports non pourvus de cellules.

23. Procédé selon la revendication 1, dans lequel au moins 99 % des cellules possèdent des propriétés de cellules souches après le début de la culture.

24. Procédé selon la revendication 1, dans lequel le nombre de cellules augmente d'au moins 50 % par rapport au nombre de cellules de départ en une semaine de culture.

25. Procédé selon la revendication 1, dans lequel le nombre de cellules augmente d'au moins 600 % par rapport au nombre de cellules de départ en une semaine de culture.

26. Procédé selon la revendication 1, dans lequel la culture des cellules débute avec une concentration de moins de 10⁶ cellules par millilitre.

27. Procédé selon la revendication 1, dans lequel les cellules sont détachées des microsupports après le prélèvement des cellules avec les microsupports.

28. Procédé selon la revendication 1, dans lequel le prélèvement des cellules est opéré aux fins du contrôle de l'obtention des propriétés de cellules souches.
